(19) Europäisches Patentamt
European Patent Office
Office européen des brevets

(11) EP 2 656 410 B1

(12) **EUROPÄISCHE PATENTSCHRIFT**

(45) Veröffentlichungstag und Bekanntmachung des Hinweises auf die Patenterteilung:
**05.07.2017 Patentblatt 2017/27**

(21) Anmeldenummer: **11788060.9**

(22) Anmeldetag: **24.11.2011**

(51) Int Cl.:
***H01L 51/54*** *(2006.01)*

(86) Internationale Anmeldenummer:
**PCT/EP2011/005917**

(87) Internationale Veröffentlichungsnummer:
**WO 2012/084115 (28.06.2012 Gazette 2012/26)**

(54) **ORGANISCHE ELEKTROLUMINESZENZVORRICHTUNG**

ORGANIC ELECTROLUMINESCENT DEVICE

DISPOSITIF ÉLECTROLUMINESCENT ORGANIQUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **23.12.2010 DE 102010055902**

(43) Veröffentlichungstag der Anmeldung:
**30.10.2013 Patentblatt 2013/44**

(73) Patentinhaber: **Merck Patent GmbH**
**64293 Darmstadt (DE)**

(72) Erfinder:
- **PAN, Junyou**
  **60320 Frankfurt am Main (DE)**
- **STOESSEL, Philipp**
  **60487 Frankfurt am Main (DE)**

(56) Entgegenhaltungen:
**US-A1- 2008 015 269**
**US-A1- 2008 054 223**
**US-A1- 2008 224 089**
**US-A1- 2010 059 740**

- **A. D. JORDAN ET AL: "Single vibronic level fluorescence. V. Pyridazine", CHEMICAL PHYSICS LETTERS, Bd. 16, Nr. 3, 15. Oktober 1972 (1972-10-15), Seiten 437-441, XP55020783, DOI: 10.1016/0009-2614(72)80395-6,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Salz, bei dem sowohl mindestens ein Kation als auch mindestens ein Anion eine Emitterverbindung oder eine Farbstoffverbindung ist, dadurch gekennzeichnet, dass eine Emitterverbindung eine fluoreszierende Emitterverbindung ist. Zudem betrifft die vorliegende Erfindung auch ein Verfahren zur Herstellung des erfindungsgemäßen Salzes, die Verwendung des Salzes in einer elektronischen Vorrichtung, sowie eine Formulierung und eine elektronische Vorrichtung, die das Salz enthält.

Elektronische Vorrichtungen, welche organische, metallorganische und/oder polymere Halbleiter enthalten, werden immer häufiger in kommerziellen Produkten verwendet oder stehen kurz vor der Markteinführung. Als Beispiele seien hier Ladungstransportmaterialien auf organischer Basis (in der Regel Lochtransporter auf Triarylamin-Basis) in Kopiergeräten und organische oder polymere Leuchtdioden (OLEDs oder PLEDs) in Anzeige- und Displayvorrichtungen oder organische Photorezeptoren in Kopierern genannt. Auch organische Solarzellen (O-SC), organische Feldeffekt-Transistoren (O-FET), organische Dünnfilm-Transistoren (O-TFT), organische Schaltelemente (O-IC), organische optische Verstärker oder organische Laserdioden (O-Laser) sind in einem fortgeschrittenen Entwicklungsstand und können in der Zukunft große Bedeutung erlangen.

Viele dieser elektronischen bzw. opto-elektronischen Vorrichtungen weisen unabhängig von dem jeweiligen Verwendungszweck folgenden allgemeinen Schichtaufbau auf, der für die jeweilige Anwendung angepasst werden kann:

(1) Substrat,
(2) Elektrode, häufig metallisch oder anorganisch, aber auch aus organischen bzw. polymeren leitfähigen Materialien;
(3) Ladungsinjektionsschicht bzw. Zwischenschicht zum Ausgleich von Unebenheiten der Elektrode ("planarisation layer"), häufig aus einem leitfähigen, dotierten Polymer,
(4) Organische Halbleiter,
(5) evtl. eine weitere Ladungstransport- bzw. Ladungsinjektions- bzw. Ladungsblockierschicht,
(6) Gegenelektrode, Materialien wie unter (2) genannt,
(7) Verkapselung.

Die obige Anordnung stellt den allgemeinen Aufbau einer opto-elektronischen Vorrichtung dar, wobei verschiedene Schichten zusammengefasst werden können, so dass im einfachsten Fall eine Anordnung aus zwei Elektroden, zwischen denen sich eine organische Schicht befindet, resultiert. Die organische Schicht erfüllt in diesem Fall alle Funktionen, einschließlich der Emission von Licht. Ein derartiges System ist beispielsweise in der WO 9013148 A1 auf der Basis von Poly-(p-phenylenen) beschrieben.

Ein Problem, das sich in einem derartigen "Dreischichtsystem" ergibt, ist jedoch die fehlende Möglichkeit, die einzelnen Bestandteile in unterschiedlichen Schichten bezüglich ihrer Eigenschaften zu optimieren, wie es beispielsweise bei SMOLEDs ("small-molecule OLEDs") durch einen mehrschichtigen Aufbau einfach gelöst ist. Eine "small molecule OLED" besteht beispielsweise aus einer oder mehreren organischen Lochinjektionsschichten, Lochtransportschichten, Emissionsschichten, Elektronentransportschichten und Elektroneninjektionsschichten sowie einer Anode und einer Kathode, wobei sich das ganze System gewöhnlich auf einem Glassubstrat befindet. Ein Vorteil einer solchen Mehrlagenstruktur besteht darin, dass verschiedene Funktionen der Ladungsinjektion, des Ladungstransports und der Emission in die verschiedenen Schichten aufgeteilt und somit die Eigenschaften der jeweiligen Schichten separat modifiziert werden können.

Das Aufbringen der Schichten in SMOLED-Vorrichtungen erfolgt gewöhnlich durch Aufdampfen in einer Vakuumkammer. Dieses Verfahren ist jedoch aufwändig und somit teuer und insbesondere für große Moleküle, wie beispielsweise Polymere, aber auch für viele kleine Moleküle, die sich unter den Aufdampfbedingungen häufig zersetzen, ungeeignet.

Vorteilhaft ist deshalb die Aufbringung von Schichten aus Lösung, wobei sowohl kleine Moleküle, als auch Oligomere oder Polymere aus Lösung prozessiert werden können.

Im herkömmlichen Verfahren zur OLED-Herstellung, sowohl durch Abscheidung aus der Gasphase oder lösungsprozessiert, ist es schwierig die Verteilung der einzelnen Komponenten zu kontrollieren. Die Komponenten verteilen sich gewöhnlich statistisch. Für einige physikalische Eigenschaften solcher Systeme ist dies unerwünscht, beispielsweise beim sogenannten "double doping" in Triplett-Systemen (siehe Kawamura, Y.; Yanagida, S.; Forrest, S.R., "Energy transfer in polymer electro phosphorescent light emitting device with single and multiple doped luminescent layers", J. Appl. Phys., 92 (1), 87 - 93, 2002). Darin wird berichtet, dass ein sehr effizientes Polymer (PHOLED) dadurch erzeugt wird, indem Poly(9-vinylcarbazol) (PVK) als Host-Molekül, welches mit einem oder mehreren phosphoreszierenden cyclometallierten Ir(III)-Komplexen dotiert ist, verwendet wird. Gewöhnlich wird angenommen, dass im Falle einer doppelten Dotierung ein Energietransfer, beispielsweise nach dem Förster-Mechanismus stattfindet.

Die Förster-Energietransferrate kann beispielsweise theoretisch nach folgender Formel dargestellt werden:

$$\Gamma_{DA} \propto 1/R^6,$$

wobei R den Abstand zwischen Donor und Akzeptor darstellt. Dieser Abstand wird gewöhnlich auch als der Förster-Radius bezeichnet. Um einen effizienten Energietransfer, z.B. gemäß Förster oder Dexter zu ermöglichen, ist es somit notwendig den Donor und Akzeptor, das heißt die beiden Emitterverbindungen bzw. Metallkomplexe so nahe wie möglich zu positionieren, vorteilhafterweise innerhalb des sogenannten Förster-Radius.

Dadurch, dass sich die beiden Emitter gewöhnlich statistisch verteilen, ist der notwendige geringe Abstand der beiden Emittermoleküle voneinander (Donor und Akzeptor) nicht im vollen Umfang gewährleistet.

US 2010/059740 A1 offenbart allgemein geladene Emitter und deren Verwendung in OLEDs und insbesondere geladene Emitter, die an eine polymere Matrix mit entgegengesetzter Ladung binden.

Die Aufgabe der vorliegenden Erfindung bestand deshalb in der Bereitstellung eines Systems, in dem mindestens zwei Emitterverbindungen den notwendigen geringen Abstand zueinander durch elektrostatische Wechselwirkung aufweisen, der für einen effizienten Energietransfer zwischen den Emittermolekülen notwendig ist.

Die vorliegende Erfindung stellt hierfür ein Salz gemäß Anspruch 1 bereit.

In anderen Worten enthält das Salz mindestens eine Kation, das eine Emitterverbindung oder eine Farbstoffverbindung ist, und ein Anion, das eine Emitterverbindung oder eine Farbstoffverbindung ist.

Unter einem Salz wird in der vorliegenden Erfindung eine ionische Verbindung aus Kationen und Anionen verstanden, deren positive und negative Ladungen sich ausgleichen bzw. neutralisieren. Das erfindungsgemäße Salz kann ein einfaches Salz aus einer Art von Kationen und einer Art von Anionen sein, es kann aber auch ein Doppelsalz sein, entweder mit zwei oder mehreren verschiedenen Kationen und einer Art Anionen, oder mit zwei oder mehreren verschiedenen Anionen und einer Art Kationen. Neben einfachen Salzen und Doppelsalzen kann das erfindungsgemäße Salz auch als Mischsalz vorliegen, indem mindestens zwei verschiedene einfache Salze oder Doppelsalze, oder ein einfaches Salz und ein Doppelsalz miteinander einen Mischkristall bilden.

Vorzugsweise ist mindestens eines der Kationen des erfindungsgemäßen Salzes eine positiv geladene Emitterverbindung oder Farbstoffverbindung und mindestens eines der Anionen eine negativ geladene Emitterverbindung oder Farbstoffverbindnung. Besonders bevorzugt ist, dass alle Ionen des erfindungsgemäßen Salzes ausgewählt sind aus Emitterverbindungen oder Farbstoffverbindungen.

Eine fluoreszierende Emitterverbindung im Sinne dieser Erfindung ist allgemein betrachtet eine Einheit, die Licht, vorzugsweise im sichtbaren Bereich, aus einem angeregten Singulett-Zustand emittiert, und eine positive oder negative Ladung aufweist. Die fluoreszierende Emitterverbindung kann in dem erfindungsgemäßen Salz als Kation oder als Anion fungieren. Besonders bevorzugt sind hierbei einfach oder zweifach positiv oder negativ geladene Kationen oder Anionen, besonders bevorzugt einfach positiv oder negativ geladene Kationen oder Anionen.

Die fluoreszierende Emitterverbindung umfasst vorzugsweise eine der folgenden Einheiten: Mono- oder polycyclische aromatische oder heteroaromatischen Ringsysteme mit 5 bis 60 aromatischen Ringatomen oder auch Tolan-, Stilben- oder Bisstyrylarylenderivate, die jeweils mit einem oder mehreren Resten R substituiert sein können. Besonders bevorzugt ist dabei der Einbau von 1,4-Phenyl, 1,4-Naphthyl, 1,4- oder 9,10-Anthryl, 1,6-, 2,7- oder 4,9-Pyrenyl, 3,9- oder 3,10-Perylenyl, 4,4'-Biphenylyl, 4,4"-Terphenylyl, 4,4'-Bi-1,1'-naphthylyl, 4,4'-Tolanyl, 4,4'-Stilbenyl, 4,4"-Bisstyrylaryl, Benzothiadiazolyl, Chinoxalinyl, Phenothiazinyl, Phenoxazinyl, Dihydrophenazinyl, Bis(thiophenyl)aryl, Oligo(thiophenyl), Phenazinyl, Rubrenyl, Pentacenyl, Squarinyl und Chinacridonyl, die vorzugsweise substituiert sind, oder vorzugsweise konjugierte Push-Pull-Systeme (Systeme, die mit Donor- und Akzeptorsubstituenten substituiert sind, oder Systeme wie Squarine oder Chinacridone, die vorzugsweise substituiert sind.

Unter einem Monostyrylamin wird eine Verbindung verstanden, die eine substituierte oder unsubstituierte Styrylgruppe und mindestens ein, bevorzugt aromatisches, Amin enthält. Unter einem Distyrylamin wird eine Verbindung verstanden, die zwei substituierte oder unsubstituierte Styrylgruppen und mindestens ein, bevorzugt aromatisches, Amin enthält. Unter einem Tristyrylamin wird eine Verbindung verstanden, die drei substituierte oder unsubstituierte Styrylgruppen und mindestens ein, bevorzugt aromatisches, Amin enthält. Unter einem Tetrastyrylamin wird eine Verbindung verstanden, die vier substituierte oder unsubstituierte Styrylgruppen und mindestens ein, bevorzugt aromatisches, Amin enthält. Die Styrylgruppen sind besonders bevorzugt Stilbene, die auch noch weiter substituiert sein können. Entsprechende Phosphine und Ether sind in Analogie zu den Aminen definiert. Unter einem Arylamin bzw. einem aromatischen Amin im Sinne dieser Erfindung wird eine Verbindung verstanden, die drei substituierte oder unsubstituierte aromatische oder heteroaromatische Ringsysteme direkt an den Stickstoff gebunden enthält. Bevorzugt ist mindestens eines dieser aromatischen oder heteroaromatischen Ringsysteme ein kondensiertes Ringsystem, bevorzugt mit mindestens 14 aromatischen Ringatomen. Bevorzugte Beispiele hierfür sind aromatische Anthracenamine, aromatische Anthracendiamine, aromatische Pyrenamine, aromatische Pyrendiamine, aromatische Chrysenamine oder aromatische Chrysendiamine. Unter einem aromatischen Anthracenamin wird eine Verbindung verstanden, in der eine Diarylaminogruppe direkt an eine Anthracengruppe gebunden ist, vorzugsweise in 9-Position. Unter einem aromatischen Anthracendiamin wird

eine Verbindung verstanden, in der zwei Diarylaminogruppen direkt an eine Anthracengruppe gebunden sind, vorzugsweise in 2,6- oder 9,10-Position. Aromatische Pyrenamine, Pyrendiamine, Chrysenamine und Chrysendiamine sind analog dazu definiert, wobei die Diarylaminogruppen am Pyren bevorzugt in 1-Position bzw. in 1,6-Position gebunden sind.

Weitere bevorzugte fluoreszierende Emitterverbindungen sind gewählt aus Indenofluorenaminen bzw. -diaminen, beispielsweise gemäß WO 2006/122630, Benzoindenofluorenaminen bzw. -diaminen, beispielsweise gemäß WO 2008/006449, und Dibenzoindenofluorenaminen bzw. - diaminen, beispielsweise gemäß WO 2007/140847.

Weitere bevorzugte fluoreszierende Emitterverbindungen sind gewählt aus Derivaten von Naphthalin, Anthracen, Tetracen, Benzanthracen, Benzphenanthren (DE 10 2009 005746.3), Fluoren, Fluoranthen, Periflanthen, Indenoperylen, Phenanthren, Perylen (US 2007/0252517 A1), Pyren, Chrysen, Decacyclen, Coronen, Tetraphenylcyclopentadien, Pentaphenylcyclopentadien, Fluoren, Spirofluoren, Rubren, Cumarin (US 4769292, US 6020078, US 2007/0252517 A1), Pyran, Oxazol, Benzoxazol, Benzothiazol, Benzimidazol, Pyrazin, Zimtsäureestern, Diketopyrrolopyrrol, Acridon und Chinacridon (US 2007/0252517 A1).

Von den Anthracenverbindungen sind besonders bevorzugt in 9,10-Position substituierte Anthracene wie z.B. 9,10-Diphenylanthracen und 9,10-Bis(phenylethynyl)anthracen. Auch 1,4-Bis(9'-ethynylanthracenyl)benzol ist ein bevorzugter Dotand. Ebenfalls bevorzugt sind Derivate von Rubren, Cumarin, Rhodamin, Chinacridon wie z.B. DMQA (= N,N'-dimethylchinacridon), Dicyanomethylenpyran wie z.B. DCM (= 4-(dicyanoethylen)-6-(4-dimethylaminostyryl-2-methyl)-4H-pyran), Thiopyran, Polymethin, Pyryliumund Thiapyryliumsalzen, Periflanthen und Indenoperylen.

Blaue Fluoreszenzemitter sind bevorzugt Polyaromaten wie z.B. 9,10-Di(2-naphthylanthracen) und andere Anthracen-Derivate, Derivate von Tetracen, Xanthen, Perylen wie z.B. 2,5,8,11-Tetra-*t*-butyl-perylen, Phenylen, z.B. 4,4'-(Bis(9-ethyl-3-carbazovinylen)-1,1'-biphenyl, Fluoren, Fluoranthen, Arylpyrene (US Ser.No. 11/097352 filed Apr.4,2005), Arylenvinylene (US 5121029, US 5130603), Bis(azinyl)imin-Bor-Verbindungen (US 2007/0092753 A1), Bis(azinyl)methenverbindungen und Carbostyryl-Verbindungen.

Weitere bevorzugte blaue Fluoreszenzemitter sind in C.H.Chen et al.: "Recent developments in organic electroluminescent materials" Macromol. Symp. 125, (1997) 1-48 und "Recent progress of molecular organic electroluminescent materials and devices" Mat. Sci. and Eng. R, 39 (2002), 143-222 beschrieben.

Weitere bevorzugte blau fluoreszierende Emitter sind die in der nicht offen gelegten Anmeldung DE 102008035413 offenbarten Kohlenwasserstoffe.

Beispiele für erfindungsgemäß einsetzbare fluoreszierende Emitterverbindungen sind der folgenden Übersicht mit den Verbindungen mit den Formeln (1) bis (48) zu entnehmen:

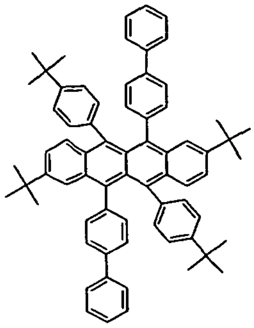

Formel (1)

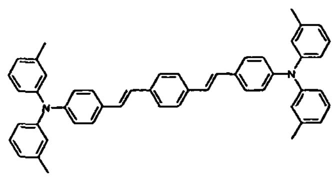

Formel (2)

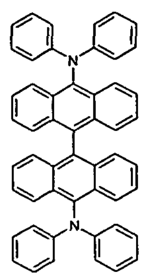

Formel (3)

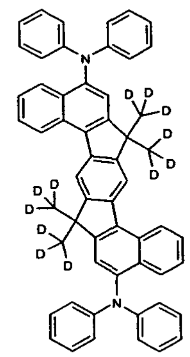

Formel (4)

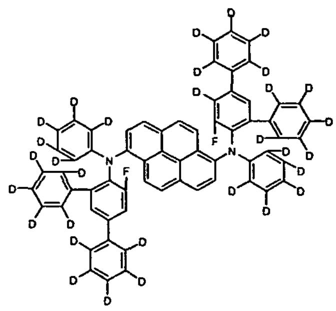

Formel (5)

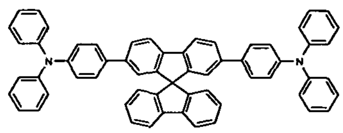

Formel (6)

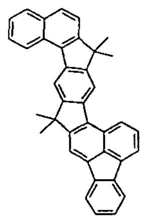

Formel (7)

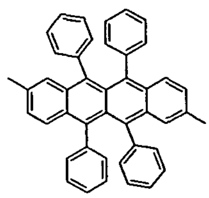

Formel (8)

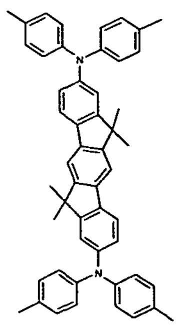

Formel (9)

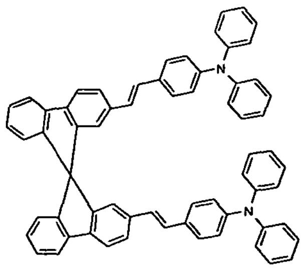

Formel (10)

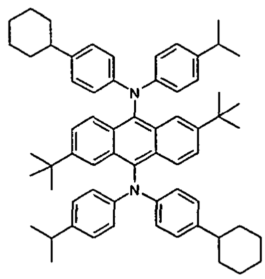

Formel (11)

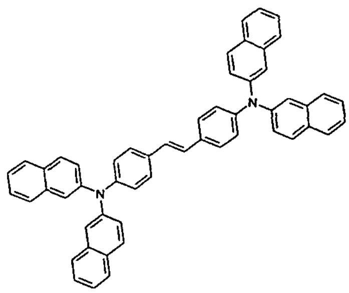

Formel (12)

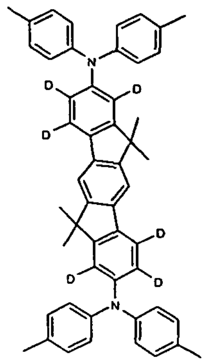

Formel (13)

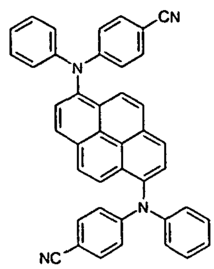

Formel (14)

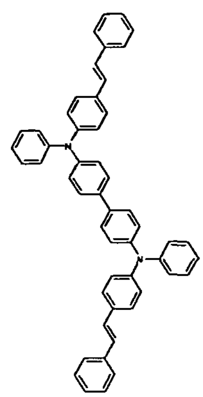

Formel (15)

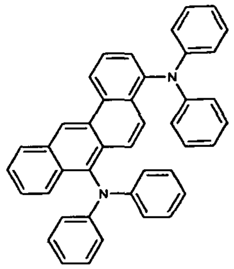

Formel (16)

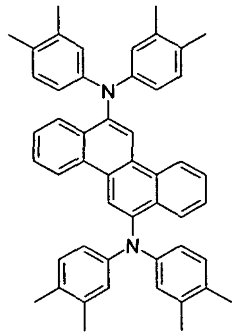

Formel (17)

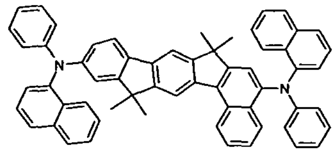

Formel (18)

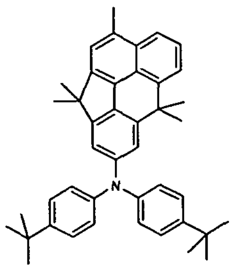

Formel (19)

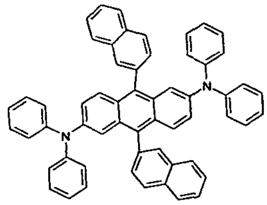

Formel (20)

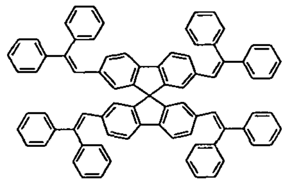

Formel (21)

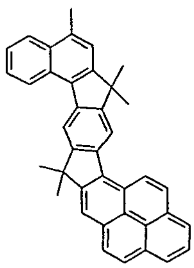

Formel (22)

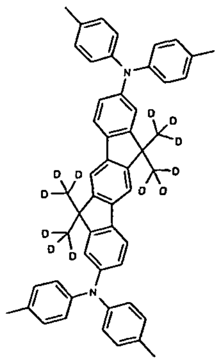

Formel (23)

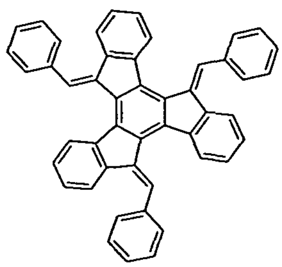

Formel (24)

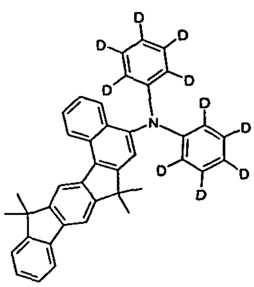

Formel (25)

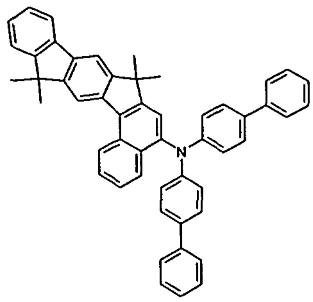

Formel (26)

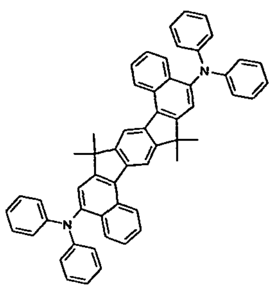

Formel (27)

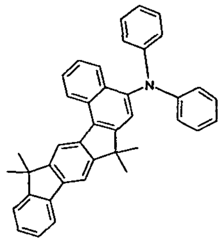

Formel (28)

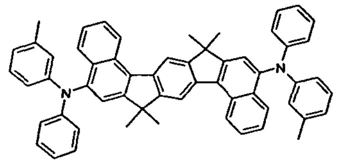

Formel (29)

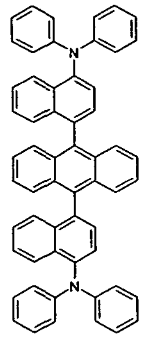

Formel (30)

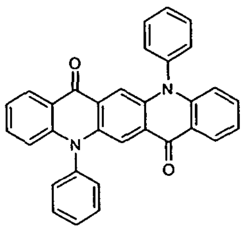

Formel (31)

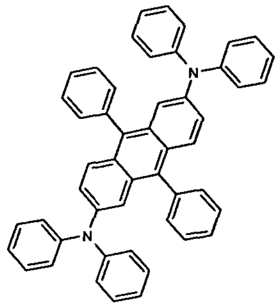

Formel (32)

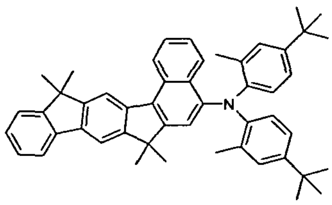

Formel (33)

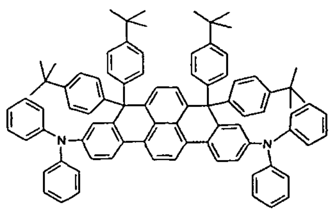

Formel (34)

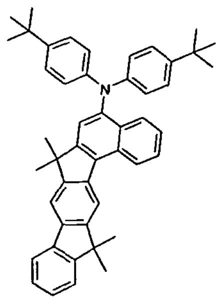

Formel (35)

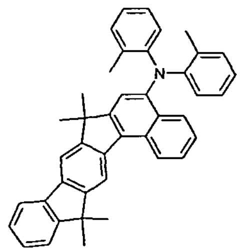

Formel (36)

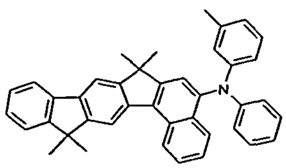

Formel (37)

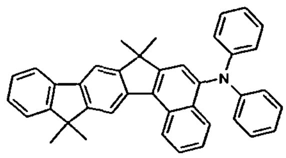

Formel (38)

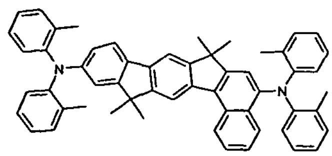

Formel (39)

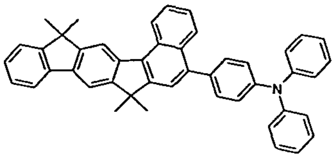
Formel (40)

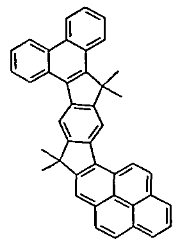
Formel (41)

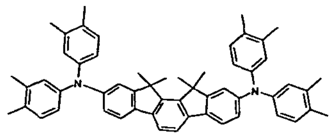
Formel (42)

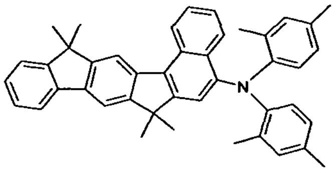
Formel (43)

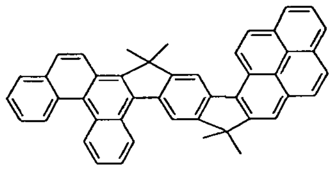
Formel (44)

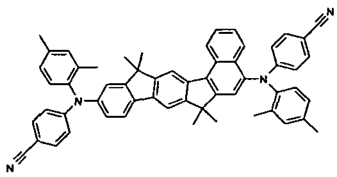
Formel (45)

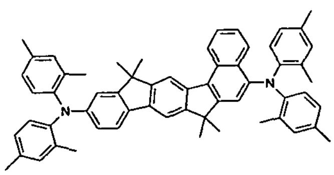
Formel (46)

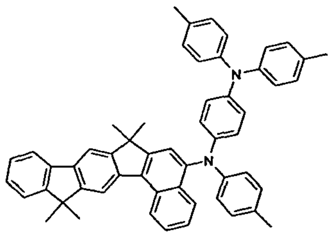
Formel (47)

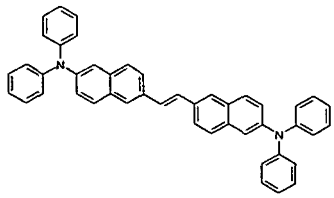
Formel (48)

Die oben genannten fluoreszierenden Emitterverbindungen sind vorzugsweise organische Emitterverbindungen enthaltend mindestens einen geladenen aliphatischen Substituenten, wobei der geladene aliphatische Substituent vorzugsweise $R_1O^-$, $R_1S^-$, $R_1C(O)^-$, $R_1R_2R_3N^+$, $R_1R_2R_3P^+$, $R_1SO_3^-$,oder eine der unten genannten Seitengruppen ist.

Geeignete organische Kationen sind ausgewählt aus der Gruppe, die Ammonium-, Phosphonium, Thiouronium- und Guanidinium Kationen umfasst, wie sie in den Formeln (49) bis (53) oder den heterocyclischen Kationen der Formeln (54) bis (81) gezeigt sind:

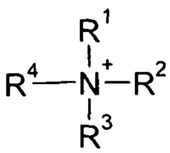
Formel (49)

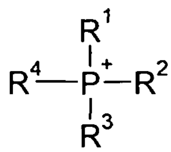
Formel (50)

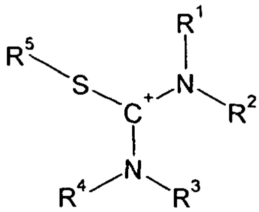
Formel (51)

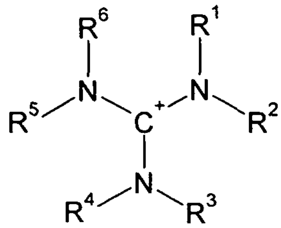
Formel (52)

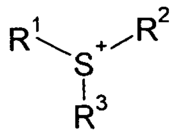

Formel (53)

wobei

$R^1$ bis $R^6$ vorzugsweise unabhängig voneinander ausgewählt sind aus der Gruppe bestehend aus geradkettigen oder verzweigten Alkylgruppen mit 1 bzw. 3 bis 20 C-Atomen, geradkettigen oder verzweigten Alkenylgruppen mit 2 bzw. 3 bis 20 C-Atomen und einer oder mehrerer nicht-konjugierten Doppelbindungen, geradkettigen oder verzweigten Alkinylgruppen mit 2 bzw. 3 bis 20 C-Atomen mit einer oder mehreren nicht-konjugierten Dreifachbindungen und einer gesättigten, teilweise gesättigten oder vollständig gesättigten Cycloalkylgruppe mit 3 bis 7 C-Atomen, die weiterhin mit einer Alkylgruppe mit 1 bis 6 C-Atomen substituiert sein kann, wobei einer oder mehrere der Vertreter aus $R^1$ bis $R^6$ teilweise oder vollständig mit einem Halogen, insbesondere mit F und/oder Cl, oder teilweise substituiert mit -OR', -CN, - C(O)OH, -C(O)NR'$_2$, -$SO_2$NR'$_2$, -$SO_2$OH,-$SO_2$X, -$NO_2$ sein können, wobei ein oder mehrere nicht-benachbarte Atome und nicht-$\alpha$-Kohlenstoffatome aus $R^1$ bis $R^6$ durch Atome oder eine Gruppe ausgewählt aus -O-, -S-, -S(O)-, -$SO_2$-, -$N^+$R'$_2^-$, -C(O)NR'-,-$SO_2$NR'-, and -P(O)R' substituiert sein können, wobei R' gleich H, eine teilweise fluorierte oder perfluorierte $C_1$- bis $C_6$-Alkylgruppe, $C_3$-bis $C_7$-Cycloalkylgruppe, ein unsubstituiertes oder substituiertes Phenyl ist, und X gleich Halogen ist;
und in Formel (49), $R^1$ bis $R^4$ gleich H sein können, mit der Maßgabe, dass wenigstens ein Vertreter aus $R^1$ bis $R^4$ nicht gleich H ist;

in Formel (50) $R^1$ bis $R^4$ unabhängig voneinander H oder NR'$_2$ sein können, wobei R' wie vorstehend definiert ist;

in Formel (51) $R^1$ bis $R^5$ H sein können;

in Formel (52) $R^1$ bis $R^6$ unabhängig voneinander H, CN und NR'$_2$ sein können, wobei R' wie oben definiert ist.

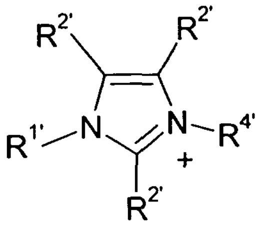

Formel (54)

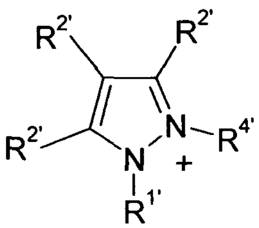

Formel (55)

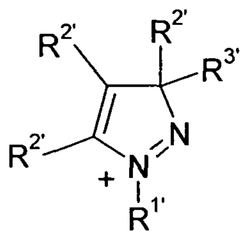

Formel (56)

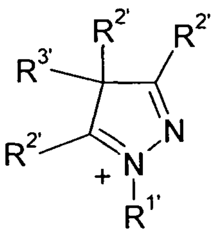

Formel (57)

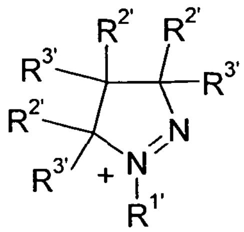

Formel (58)

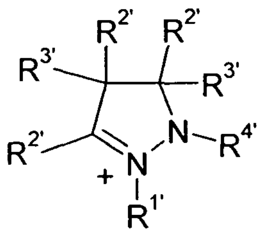

Formel (59)

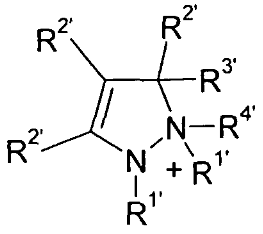

Formel (60)

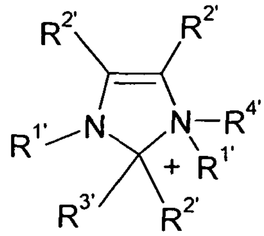

Formel (61)

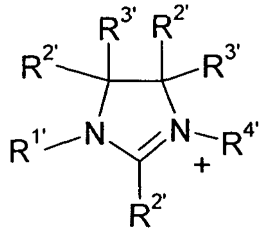

Formel (62)

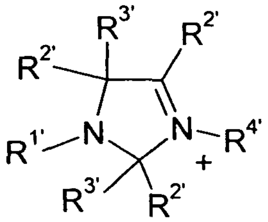

Formel (63)

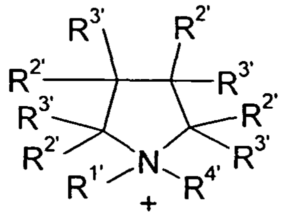

Formel (64)

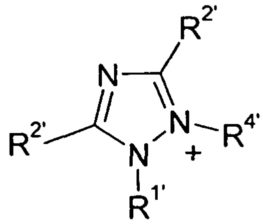

Formel (65)

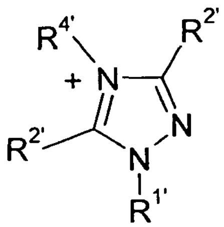

Formel (66)

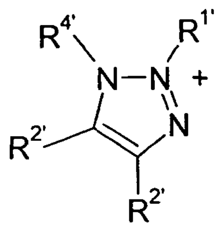

Formel (67)

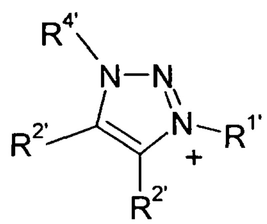

Formel (68)

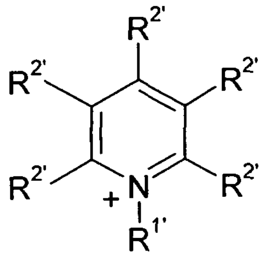

Formel (69)

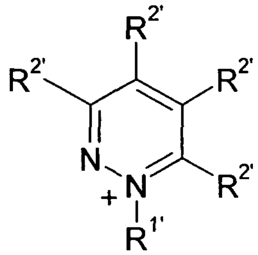

Formel (70)

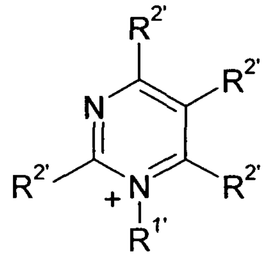

Formel (71)

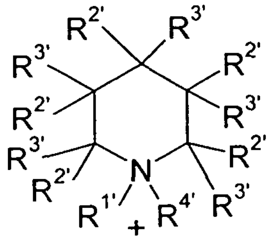

Formel (72)

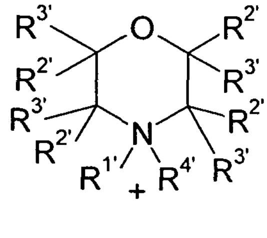

Formel (73)

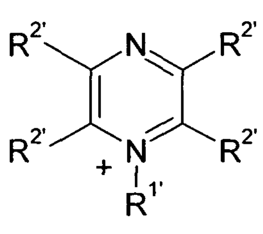

Formel (74)

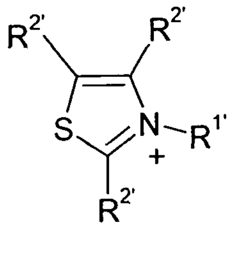

Formel (75)

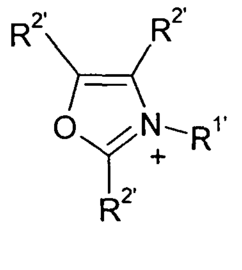

Formel (76)

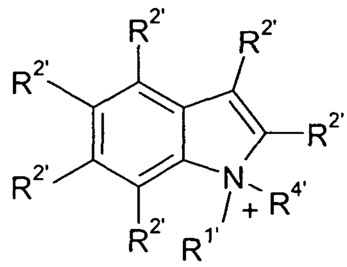

Formel (77)

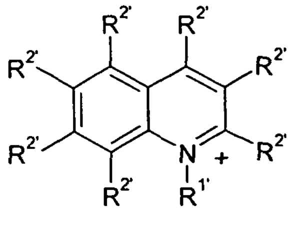

Formel (78)

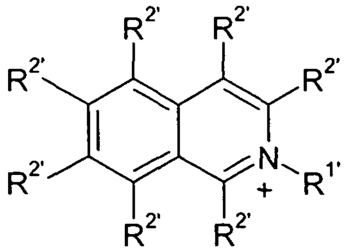

Formel (79)

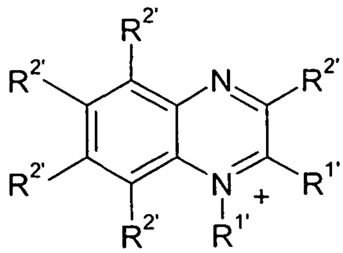

Formel (80)

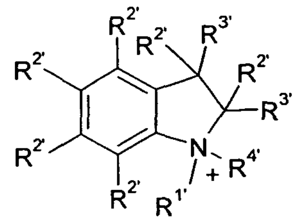

Formel (81)

In den Formeln (54) bis (81) sind die Reste $R^{1'}$ to $R^{4'}$ unabhängig voneinander aus der Gruppe ausgewählt, die aus Folgendem besteht: H, CN, einer geradkettigen oder verzweigten Alkylgruppe mit 1 bzw. 3 bis 20 C-Atomen, einer geradkettigen oder verzweigten Alkenylgruppe mit 2 bzw. 3 bis 20 C-Atomen mit einer oder mehrerer konjugierten Doppelbindung, einer geradkettigen oder verzweigten Alkinylgruppe mit 2 bzw. 3 bis 20 C-Atomen mit einer oder mehrerer nicht-konjugierten Dreifachbindungen, einer teilweise oder vollständig gesättigten Cycloalkylgruppe mit 3 bis 7 C-Atomen, die mit Alkylgruppen mit 1 bis 6 C-Atomen substituiert sein kann, einer gesättigten oder teilweise oder vollständig ungesättigten Heteroarylgruppe, Heteroaryl-$C_1$-$C_6$-alkylgruppe oder Alkyl-$C_1$-$C_6$-alkylgruppe, worin die Substituenten $R^{1'}$, $R^{2'}$, $R^{3'}$ und/oder $R^{4'}$ zusammen einen Ring bilden können, worin einer oder mehrere der Substituenten aus $R^{1'}$ bis $R^{4'}$ teilweise oder vollständig mit Halogen, vorzugsweise mit F und/oder Cl, und -OR', -CN, -C(O)OH, - C(O)$NR'_2$, -$SO_2NR'_2$, -C(O)X, -$SO_2OH$, -$SO_2X$,-$NO_2$ substituiert sein kann, worin die Substituenten $R^{1'}$ und $R^{4'}$ nicht mit beide gleichzeitig mit Halogen substituiert sind, wobei einer oder mehrere der Kohlenstoffatome der Substituenten $R^{1'}$ und $R^{2'}$, die nicht benachbart zueinander oder an einem Heteroatom gebunden sind, durch eine Einheit substituiert sein können, die aus der Gruppe ausgewählt ist, die aus -O-, - S-, -S(O)-, -$SO_2$-, -$N^+R'_2$-, -C(O)NR'-, -$SO_2$NR'- und -P(O)R'- besteht, worin R' gleich H, eine unsubstituierte, teilweise oder vollständig mit F substituierte Alkylgruppe mit 1 bis 6 C-Atomen, Cycloalkylgruppe mit 3 bis 7 C-Atomen oder unsubstituiertes oder substituiertes Phenyl ist, und X gleich Halogen ist.

Besonders bevorzugt ist $R^{2'}$ ausgewählt aus -OR',-$NR'_2$, -C(O)OH, - C(O)$NR'_2$, -$SO_2NR'_2$) -$SO_2OH$, - $SO_2X$ und -$NO_2$.

Weiterhin besonders bevorzugte kationische Gruppen umfassen ein Kation mit einer Struktur der Formel (82). Diese umfassen ein N,N,N-Trimethylbutyl ammoniumion, N-Ethyl-N,N-dimethyl-propylammoniumion, N-Ethyl-N,N-dimethylbutylammoniumion, N,N,-Dimethyl-N-propylbutyl-ammoniumion, N-(2-Methoxyethyl)-N,N-dimethylethylammoniumion, 1-Ethyl-3-methyl imidazoliumion, 1-Ethyl-2,3-dimethylimidazoliumion, 1-Ethyl-3,4-dimethyl imidazoliumion, 1-Ethyl-2,3,4-trimethylimidazoliumion, 1-Ethyl-2,3,5-trimethyl imidazoliumion, N-Methyl-N-propylpyrrolidiniumion, N-Butyl-N-methyl pyrrolidiniumion, N-sec-Butyl-N-methylpyrrolidiniumion, N-(2-Methoxyethyl)-N-methylpyrrolidiniumion, N-(2-Ethoxyethyl)-N-methylpyrrolidiniumion, N-Methyl-N-propylpiperidiniumion, N-Butyl-N-methylpipridiniumion, N-sec-Butyl-N-methylpiperidiniumion, N-(2-Methoxyethyl)-N-methylpiperidiniumion und N-(2-Ethoxyethyl)-N-methylpiperidinium-

ion.

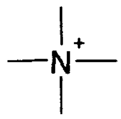

Formel (82)

Ganz besonders bevorzugt ist ein N-Methyl-N-propylpiperidiniumion.

Eine besonders bevorzugte ionische Gruppe ist eine Gruppe, die dem erfindungsgemäßen Salz eine gute Löslichkeit in einem gewöhnlichen Lösungsmitte wie Toluol, Anisol und Chloroform verleiht. Diese Gruppe is vorzugsweise eine Gruppe ausgewählt aus: Methyltrioctylammoniumtrifluoromethanesulfonat (MATS), 1-Methyl-3-octylimidazoliumoctylsulfat, 1-Butyl-2,3-dimethylimidazoliumoctylsulfat, 1-Octadecyl-3-methylimidazol-iumbis(trifluoromethylsulfonyl)imid, 1-Octadecyl-3-methylimidazolium-tris(pentafluoroethyl)trifluorophosphat, 1,1-Dipropylpyrrolidiniumbis-(trifluoromethylsulfonyl)imid, Trihexyl(tetradecyl)phosphoniumbis(1,2-bezenediolato(2-)-O,O')borat und N,N,N',N',N',N'-Pentamethyl-N'-propylguanidiniumtrifluoromethanesulfonat.

Weiterhin bevorzugte kationische Gruppen sind aus den Verbindungen der folgenden allgemeinen Formeln (83) bis (88) ausgewählt:

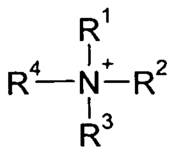

Formel (83)

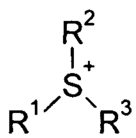

Formel (84)

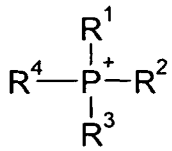

Formel (85)

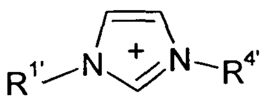

Formel (86)

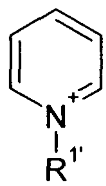

Formel (87)

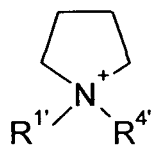

Formel (88)

$R^1$ bis $R^4$ in den Formeln (83) bis (88) sind definiert wie in den Formeln (49), (53), und (50), und $R^{1'}$ und $R^{4'}$ wie in den Formeln (54), (69) und (64).

Geeignete anionische Seitengruppen können ausgewählt werden aus $[HSO_4]^-$, $[SO_4]^{2-}$, $[NO_3]^-$,$[BF_4]^-$, $[(R)BF3]^-$, $[(R)_2BF_2]^-$, $[(R)_3BF]^-$, $[(R)_4B]^-$, $[B(CN)_4]^-$, $[PO_4]^{3-}$, $[HPO_4]^{2-}$, $[H_2PO_4]^-$, $[Alkyl\text{-}OPO_3]^{2-}$, $[(Alkyl\text{-}O)_2PO_2]^-$, $[Alkyl\text{-}PO_3]^{2-}$, $[RPO_3]^{2-}$, $[(Alkyl)_2PO_2]^-$ $[(R)_2PO_2]^-$, $[R_FSO_3]^-$, $[HOSO_2(CF_2)_nSO_2O]^-$, $[OSO_2(CF_2)_nSO_2O]^{2-}$, $[Alkyl\text{-}SO_3]^-$, $[HOSO_2(CH_2)_nSO_2O]^-$, $[OSO_2(CH_2)_nSO_2O]^{2-}$, $[Alkyl\text{-}OSO_3]^-$, $[Alkyl\text{-}C(O)O]^-$, $[HO(O)C(CH_2)_nC(O)O]^-$, $[RC(O)O]^-$, $[HO(O)C(CF_2)_nC(O)O]^-$, $[O(O)C(CF_2)_nC(O)O]^{2-}$, $[(RSO_2)_2N]^-$, $[(FS_2)_2N]^-$, $[((R)_2P(O))_2N]^-$, $[(RSO_2)_3C]^-$, $[(FSO_2)_3C]^-$, wobei n = 1 bis 8; R eine Alkylgruppe, Arylgruppe oder Alkylarylgruppe ist, die optional fluoriert oder perfluoriert sein kann.

Die oben erwähnten Alkylgruppen können aus geradkettigen oder verzweigten Alkylgruppen mit 1 bzw. 3 bis 20 C-Atomen, vorzugsweise mit 1 bis 14 C-Atomen und besonders bevorzugt 1 bis 4 C-Atomen ausgewählt werden. Vorzugsweise bedeutet $R_F$ gleich $CF_3$, $C_2F_5$, $C_3F_7$ oder $C_4F_9$.

Bevorzugte anionische Gruppen sind: $PF_6^-$, $[PF_3(C_2F_5)_3]^-$, $[PF_3(CF_3)_3]^-$, $BF_4^-$, $[BF_2(CF_3)_2]^-$, $[BF_2(C_2F_5)_2]^-$, $[BF_3(CF_3)]^-$, $[BF_3(C_2F_5)]^-$, $[B(COOCOO)_2^-$ ($BOB^-$), $CF_3SO_3^-$ (Tf), $C_4F_9SO_3(Nf^-)$, $[(CF_3SO_2)_2N]^-(TFSI^-)$, $[(C_2F_5SO_2)_2N]^-$ ($BETI^-$), $[(CF_3SO_2)(C_4F_9SO_2)N]^-$, $[(CN)_2N]^-(DCA^-)$, $[CF_3SO_2]_3C]^-$ und $[(CN)_3C]^-$.

Alle genannten geladenen aliphatischen Substituenten sind kovalent mit der Emitterverbindung verknüpft, vorzugsweise indem eines der Wasserstoffatom oder Fluoratome des Substituenten nicht vorhanden und an dieser Stelle die Bindung zu der Emitterverbindung stattfindet. Im Falle, dass die geladene Seitengruppe keine aliphatische Einheit aufweist, ist vorzugsweise ein Substituent des ladungstragenden Zentralatoms nicht vorhanden und an dieser Stelle die Bindung zu der Emitterverbindung geknüpft.

Die Polarität und die gesamte Ladung der Emitterverbindung kann durch die Wahl der Art und der Zahl der

Substituenten kontrolliert werden.

Bevorzugte Beispiele für die fluoreszierende Emitterverbindung, die eine geladene Gruppe trägt, sind der folgenden Übersicht (Formeln (89) bis (98)) zu entnehmen:

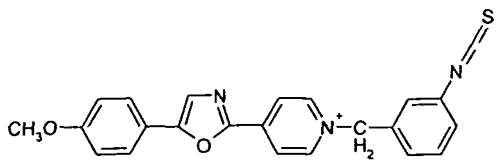

Formel (89)

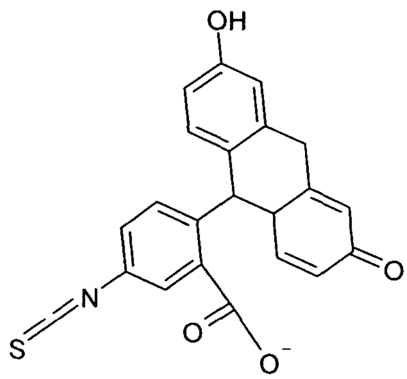

Formel (90)

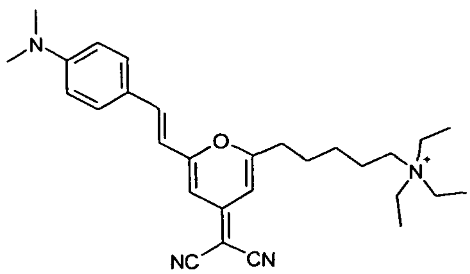

Formel (91)

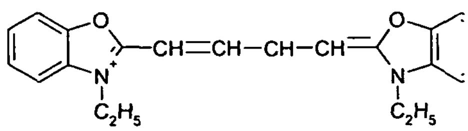

Formel (92)

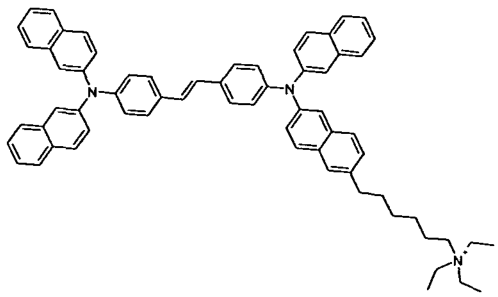

Formel (93)

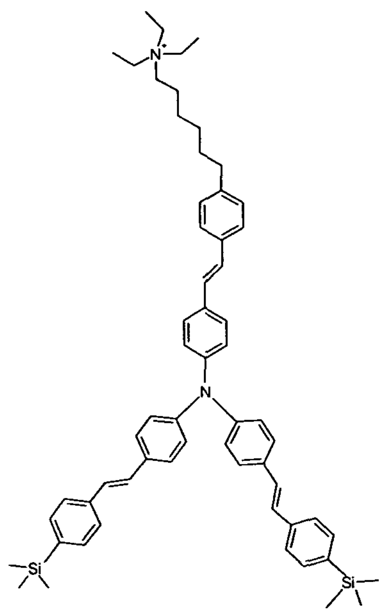

Formel (94)

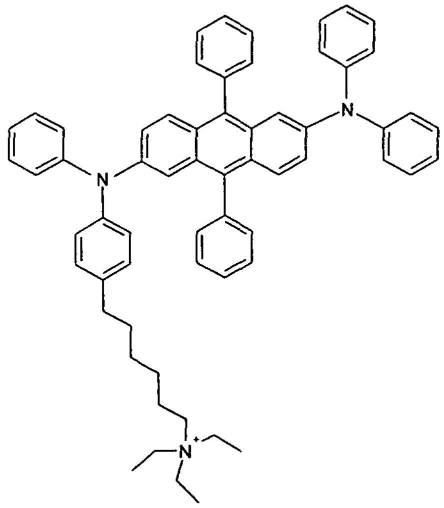

Formel (95)

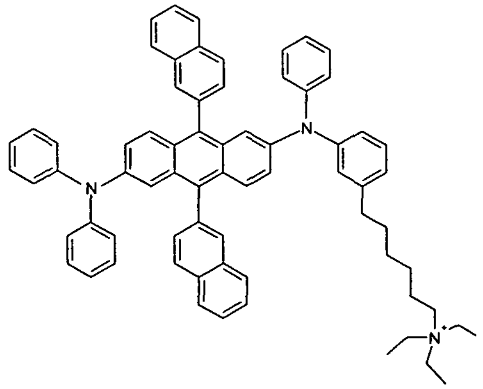

Formel (96)

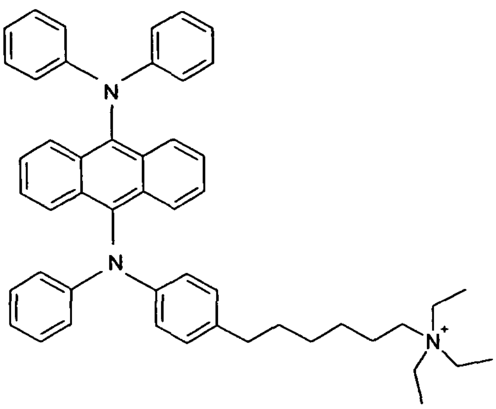

Formel (97)

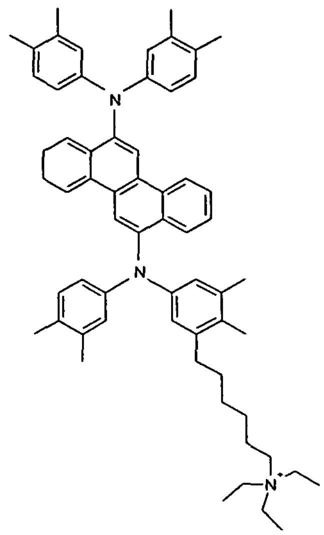

Formel (98)

Weitere Beispiele für geladene fluoreszierende Emitterverbindungen, die als Ionen im erfindungsgemäßen Salz bzw. System eingesetzt werden, können dem von Ulrich Brackmann (Lamda Physik) erstellten Katalog "Lambdachrome - Laser Dyes" entnommen werden können; unter diesen sind die folgenden erfindungsgemäß bevorzugt: DASPI, DASBTI, DMETCI, DOCI, Rhodamine 110, Rhodamine 19, Rhodamine 101, DQOCI, DQTCI, DTCI, Malachit Green, Rhodamine B, DCI-2, DODCI, DTDCI, DDI, Rhodamine 19, Cresyl Violet, Nile Blue, Oxazine 4, Rhodamine 700, Pyridine 1, Oxazine 170, Oxazine 1, Oxazine 750, Pyridine 2, HIDCI, Cryptocyanine, Styryl 6, Styryl 8, Pyridine 4, Methyl-DOTCI und Styryl 11.

Das erfindungsgemäße Salz weist durch die ionische Bindung der mindestens zwei Emitterverbindungen oder Farbstoffverbindungen hervorragende Energietransferraten zwischen den zwei Emitterzentren oder zwischen dem Emitterzentrum und dem Absorptionszentrum auf.

Unter dem Begriff "Energietransfer" versteht man in der vorliegenden Erfindung einen physikalischen Prozess, bei dem Energie eines angeregten Farbstoffs (Donor) strahlungsfrei auf einen zweiten Farbstoff (Akzeptor) übertragen wird, wie beispielsweise nach Förster (siehe T. Förster, "Zwischenmolekulare Energiewanderung und Fluoreszenz", Ann. Physic. 437, 1948, 55) oder Dexter (siehe D. L. Dexter, J. Chem. Phys., (1953) 21, 836).

In der vorliegenden Erfindung fungiert also eine Emitterverbindung vorzugsweise als Donor, und eine andere Emitterverbindung oder Farbstoffverbindung als Akzeptor im Sinne des genannten Energietransfers.

In anderen Worten kann die vorliegende Erfindung auch als ein System beschrieben werden, das eine Mehrzahl von verschiedenen geladenen Emitterverbindungen $E_i(n_j)$ oder Farbstoffverbindungen umfasst,

wobei die verwendeten Symbole und Indices die folgenden Bedeutungen aufweisen:

E(n) ist eine Emitterverbindung oder Farbstoffverbindung mit der Ladung n;

n ist eine natürliche Zahl, die nicht gleich null ist; bevorzugt ist n gleich -2, -1, 1 und 2;

i ist ein ganze Zahl, die größer oder gleich 2 ist, und die die Anzahl nicht gleicher in der Mischung vorkommender Emitterverbindungen/Farbstoffverbindungen angibt; stärker bevorzugt ist i gleich 2 oder 3;

j ist eine ganze Zahl, die größer oder gleich 2 ist, und die die Anzahl unterschiedlicher Ladungen der in der Mischung vorkommenden Emitterverbindungen/Farbstoffverbindungen angibt; stärker bevorzugt ist j gleich 2;

wobei die Summe der Ladungen aller in der Mischung vorkommenden Emitterverbindungen/Farbstoffverbindungen gleich null ist; und wenigstens eine der geladenen Emitterverbindungen eine fluoreszierende Emitterverbindung ist.

Die Farbstoffverbindung kann auch eine Metall-Ligand-Koordinationsverbindung, die nicht als emittierende Verbindung, sondern als absorbierende Verbindung fungiert, und insbesondere als Akzeptor im Sinne des genannten Energietransfers. D.h. unter einer Farbstoffverbindung versteht man keinen Stoff der durch Anregung Licht emittiert, sondern der von eingestrahltem Licht einen Teil absorbiert und den anderen Teil "übrig" lässt, der dann in Form einer anderen Farbe sichtbar ist. Generell eignen sich alle Farbstoff-Metall-Ligand-Koordinationsverbindungen, wie sie gemäß dem Stand der Technik für "dyesensitized solar cells (DSSCs)" verwendet werden und wie sie dem Fachmann auf dem Gebiet der DSSCs sind. In dieser Ausführungsform wird vorzugsweise eineFarbstoff-Metall-Ligand-Koordinationsverbindungen aus Polypyridyl Komplexen von Übergangsmetallen, vorzugsweise Ruthenium, Osmium und Kupfer ausgewählt. In einer bevorzugten Ausführungsform hat die Farbstoff-Metall-Ligand-Koordinationsverbindungen die allgemeine Struktur $ML_2(X)_2$, wobei L bevorzugt 2,2'-Bipyridyl-4,4'-dicarbonsäure ist, M ein Übergangsmetall, vorzugsweise eines aus Ru, Os, Fe, V und Cu ist, und X aus Halogen-, Cyanid, Thiocyanat, Acetylacetonat, Thiocarbamat oder Wasser ausgewählt ist. Solche Farbstoff-Metall-Ligand-Koordinationsverbindungen sind zum Beispiel in "The Journal of Physical Chemistry C 2009, 113, 2966-2973", US 2009000658, WO 2009/107100, WO 2009/098643, US 6245988, WO 2010/055471, JP 2010/084003, EP 1622178, WO 98/50393, WO 95/29924, WO 94/04497, WO 92/14741, WO 91/16719 offenbart.

Ein Beispiel für eine Farbstoffeverbindung ist:

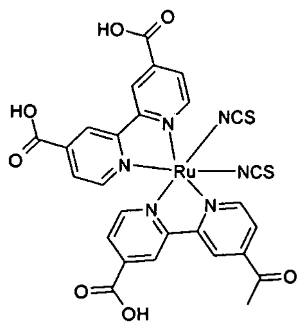

Formel (99)

In einer bevorzugten Kombination ist E1 eine Farbstoffverbindung und E2 eine fluoreszierende Emitterverbindung, wobei der Emissionsbereich von E2 mit dem Absorptionsbereich von E1 überlappt.

In einer weiteren Ausführungsform der vorliegenden Erfindung sind vorzugsweise alle Emitterverbindungen des erfindungsgemäßen Salzes oder Systems fluoreszierende Emitterverbindungen.

Es is auch möglich, dass eine der Emitterverbindungen des Salzes oder Systems eine phosphoreszierende Emitterverbindung. In diesem Fall fungiert die fluoreszierende Emitterverbindung vorzugsweise als Akzeptor, und die phosphoreszierende Emitterverbindung als Donor im Sinne des genannten Energietransfers, so dass vorzugsweise die sogenannte "Phosphorescence sensitized fluorescence" (sowie von Baldo, et al., in Nature (London) 403[6771], 750-753. 2000 beschrieben wurde) auftritt, wobei die Fluoreszenzeigenschaften der fluoreszierenden Emitterverbindung durch die Energieübertragung erhöht wird. In diesem Fall kann man auch die Energie-Niveaus von beiden Emitterverbindungen so justieren, dass vorzugsweise Extrafluoreszenz (sowie von Segal, et al., in Nat.Mater. 6[5], 374-378. 2007. beschrieben wurde) auftritt, d.h. beispielsweise OLEDs mit einer höheren Effizienz bekommen.

Unter einer phosphoreszierenden Emitterverbindung wird eine Verbindung verstanden, die Lumineszenz aus einem angeregten Zustand mit höherer Spinmultiplizität zeigt, also ein Spinzustand > 1, wie beispielsweise aus einem

angeregten Triplett-Zustand (Triplett-Emitter), aus einem MLCT-Mischzustand oder einem Quintett-Zustand (Quintett-Emitter), und eine positive oder negative Ladung aufweist. Als phosphoreszierende Emitterverbindung eignen sich insbesondere Verbindungen, die bei geeigneter Anregung Licht, vorzugsweise im sichtbaren Bereich, emittieren und außerdem mindestens ein Atom der Ordnungszahlen > 38 und < 84, besonders bevorzugt > 56 und < 80 enthalten. Bevorzugt werden als Phosphoreszenz-Emitter Verbindungen, die Kupfer, Molybdän, Wolfram, Rhenium, Ruthenium, Osmium, Rhodium, Iridium, Palladium, Platin, Silber, Gold oder Europium enthalten, insbesondere Verbindungen, die Iridium, Platin oder Kupfer enthalten. Beispiele der oben beschriebenen Emitterverbindungen können den Anmeldungen WO 00/7065, WO 01/41512, WO 02/02714, WO 02/15645, EP 1191613, EP 1191612, EP 1191614, WO 2005/033244 entnommen werden. Generell eignen sich alle geladenen phosphoreszierenden Komplexe, wie sie gemäß dem Stand der Technik für phosphoreszierende OLEDs verwendet werden und wie sie dem Fachmann auf dem Gebiet der organischen Elektrolumineszenz bekannt sind.

Beispiele für phosphoreszierende Emitterverbindungen sind der Übersicht (Formeln (132) bis (188)) zu entnehmen:

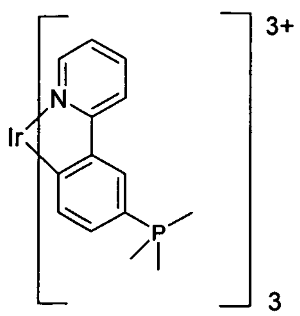

Formel (132)

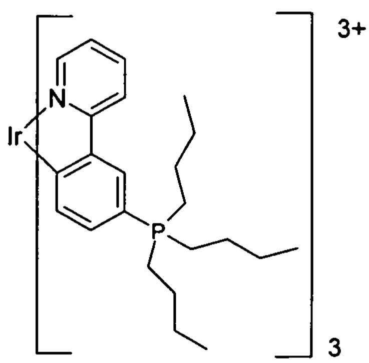

Formel (133)

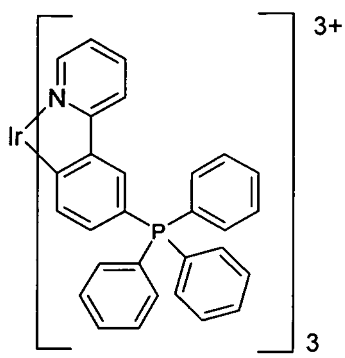

Formel (134)

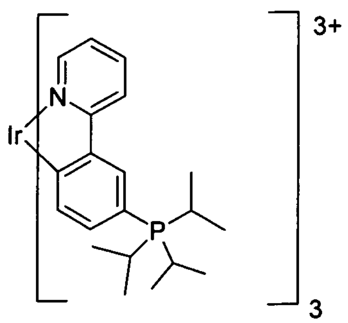

Formel (135)

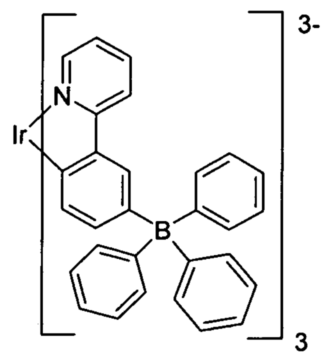

Formel (136)

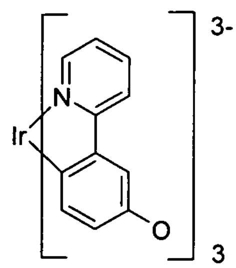

Formel (137)

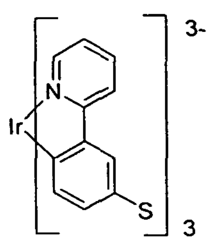

Formel (138)

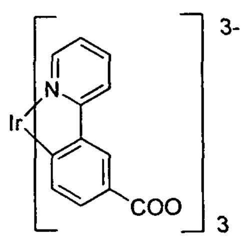

Formel (139)

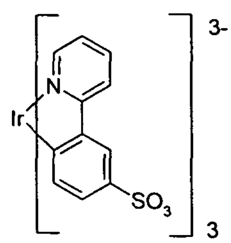

Formel (140)

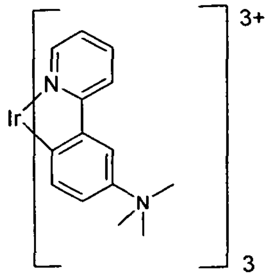

Formel (141)

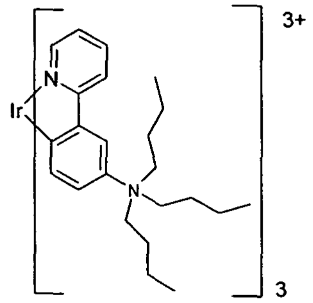

Formel (142)

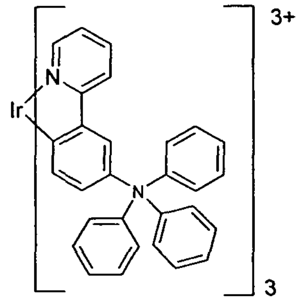

Formel (143)

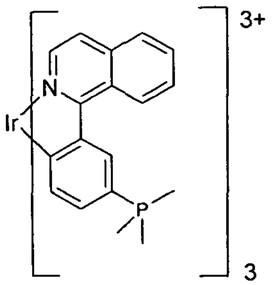

Formel (144)

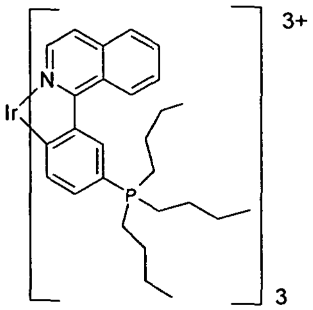

Formel (145)

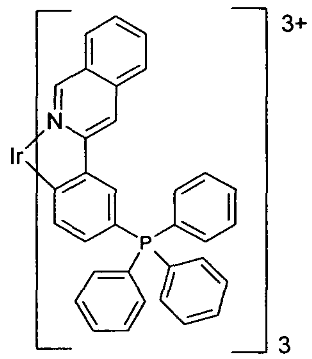

Formel (146)

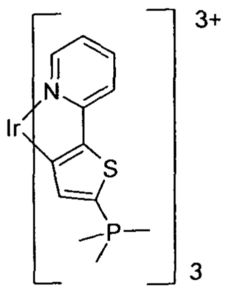

Formel (147)

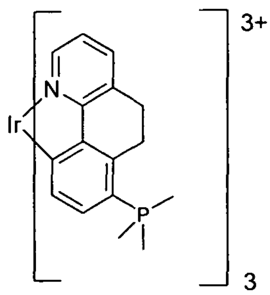

Formel (148)

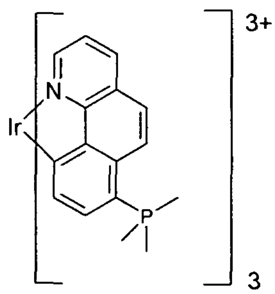

Formel (149)

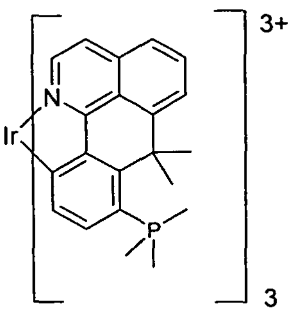

Formel (150)

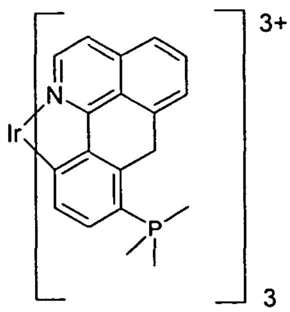

Formel (151)

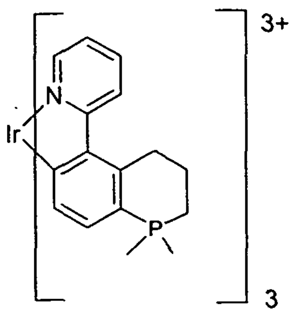

Formel (152)

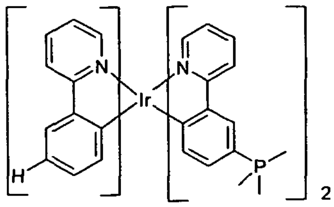

Formel (153)

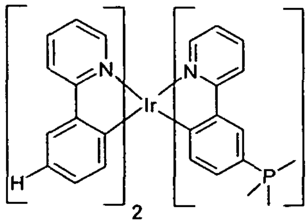

Formel (154)

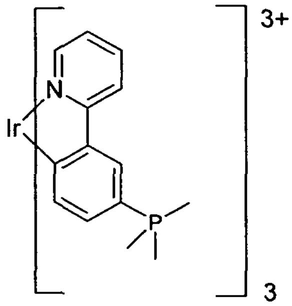

Formel (155)

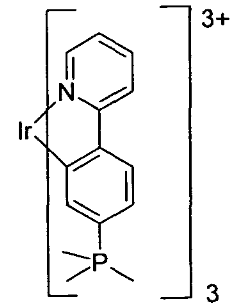

(25) Formel (156)

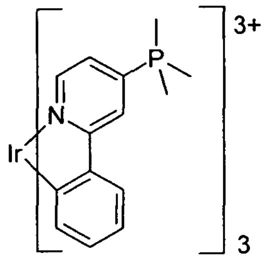

Formel (157)

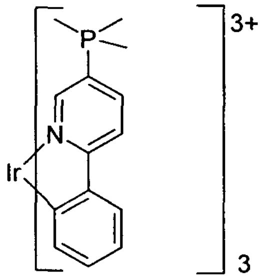

Formel (158)

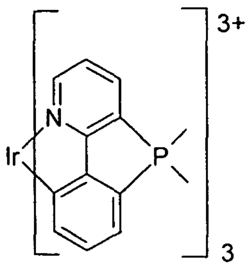

Formel (159)

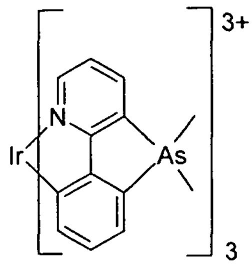

Formel (160)

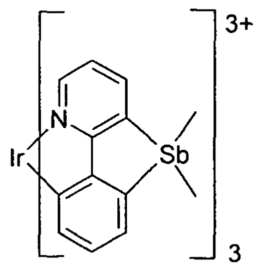

Formel (161)

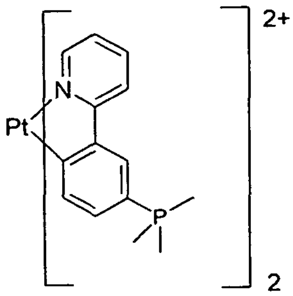

Formel (162)

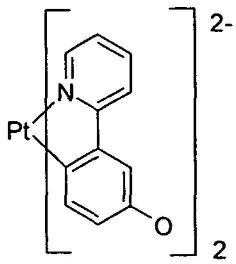

Formel (163)

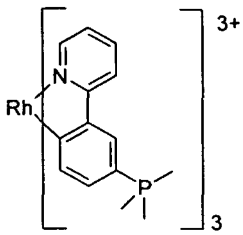

Formel (164)

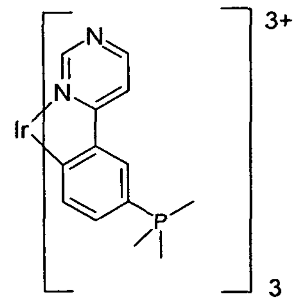

Formel (165)

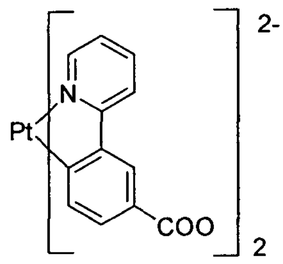

Formel (166)

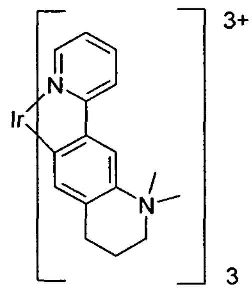

Formel (167)

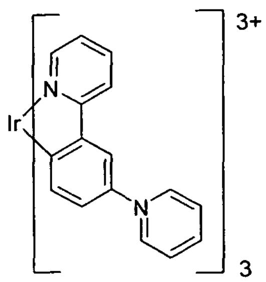

Formel (168)

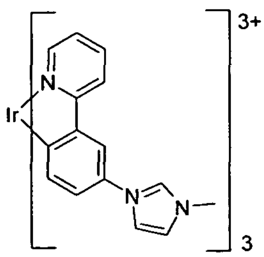

Formel (169)

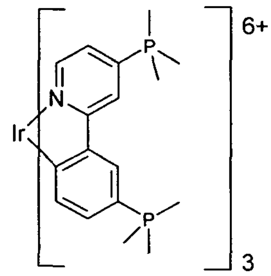

(39) Formel (170)

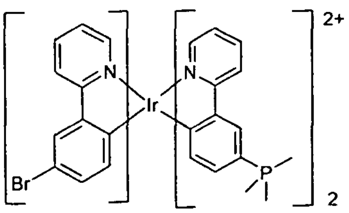

Formel (171)

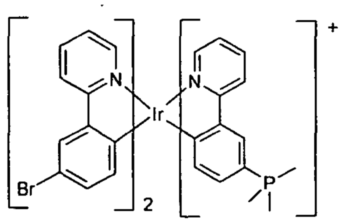

Formel (172)

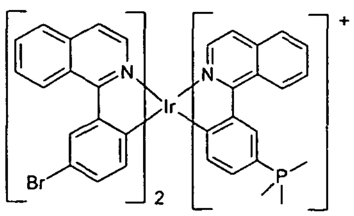

Formel (173)

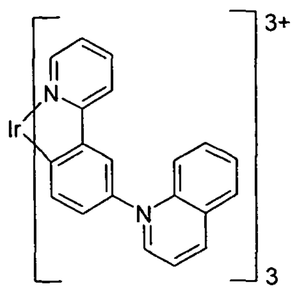

Formel (174)

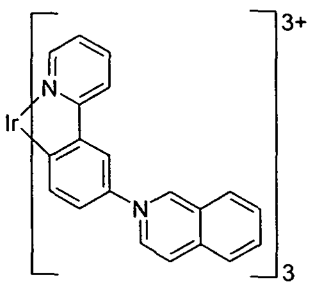

Formel (175)

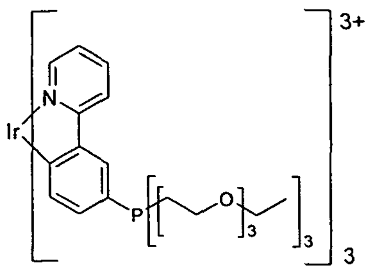

Formel (176)

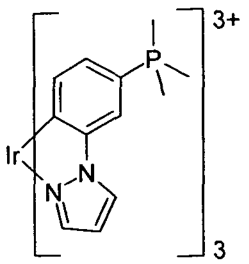

Formel (177)

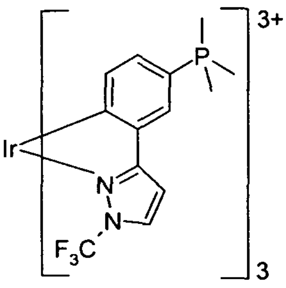

Formel (178)

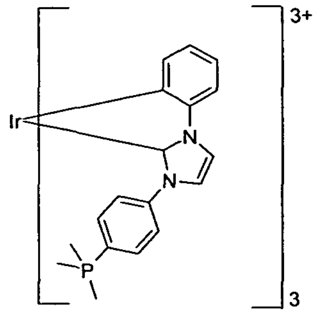

Formel (179)

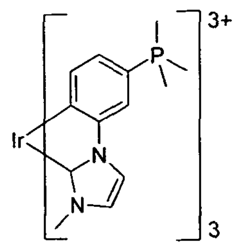

Formel (180)

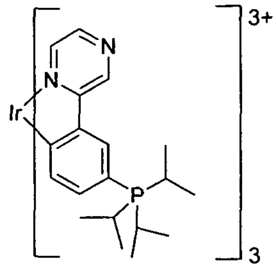

Formel (181)

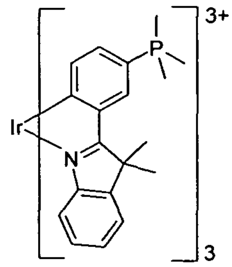

Formel (182)

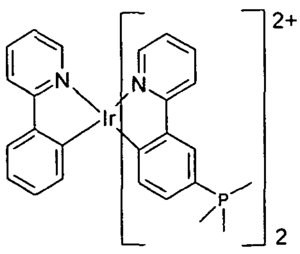

Formel (183)

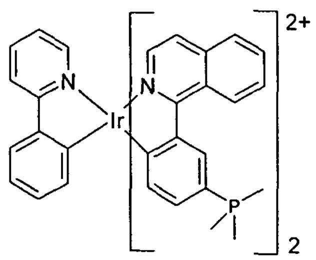

Formel (184)

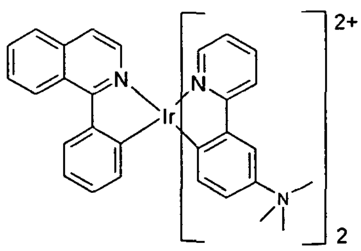

Formel (185)

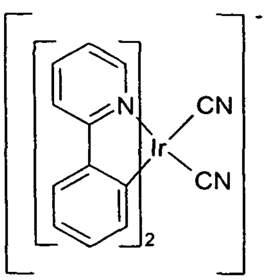

Formel (186)

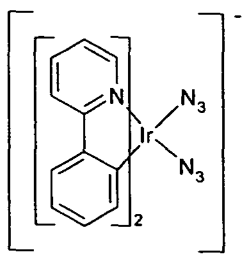

Formel (187)

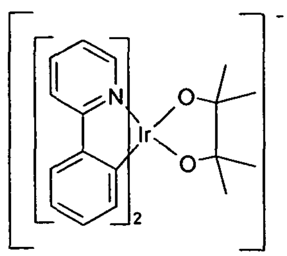

Formel (188)

Des Weiteren ist es bevorzugt, dass das erfindungsgemäße Salz oder System in der Form $[E_1]^{+m}[E_2]^{-m}$ vorliegt, wobei $[E_1]^{+m}$ eine Emitterverbindung mit der Ladung +m ist und $[E_2]^{-m}$ eine Emitterverbindung mit der Ladung -m ist, wobei m gleich 1 oder 2 ist. Besonders bevorzugt ist, dass m in diesem Fall gleich 1 ist.

$[E_1]^{-m}$ kann hierbei eine phosphoreszierende Emitterverbindung $T^{-m}$ und $[E_2]^{+m}$ eine fluoreszierende Emitterverbindung $S^{+m}$ sein, oder $[E_1]^{+m}$ und $[E_2]^{-m}$ sind fluoreszierende Emitterverbindungen $S_1^{+m}$ und $S_2^{-m}$. Auch hier ist es bevorzugt, dass m gleich 1 ist.

Des Weiteren ist es bevorzugt, dass die Emitterverbindungen $[E_1]^{-m}$ und $[E_2]^{+m}$ eine Energie "Offset"-Struktur des Typs II aufweisen, bei der das HOMO (höchstes besetztes Molekülorbital) einer ersten Emitterverbindung unterhalb des HOMO der zweiten Emitterverbindung und das LUMO (niedrigstes unbesetztes Molekülorbital) der ersten Emitterverbindung unterhalb des LUMO der zweiten Emitterverbindung liegt, aber oberhalb des HOMO der zweiten Emitterverbindung.In anderen Worten ausgedrückt hat eines der Ionen sowohl das niedrigeste Oxidationspotential, als auch das niedrigste negative Reduktionspotential. Auf diese Weise wird der gewünschte Energietransferzustand am effizientesten ermöglicht. Die Höhe der Energien von HOMO und LUMO können experimentell durch cyclovoltammetrische Messungen, oder näherungsweise theoretisch durch quantenmechanische Modellberechnungen bestimmt werden.

In einer weiteren Ausführungsform der vorliegenden Erfindung liegt das erfindungsgemäße Salz oder System in der Form $[E_3]^k[E_1]^p[E_2]^k$ vor, wobei $[E_1]^p$ eine Emitterverbindung mit der Ladung p ist und $[E_2]^k$ und $[E_3]^k$ Emitterverbindungen mit der Ladung k sind, und $p+2k=0$ ist. Der Index k ist vorzugsweise gleich $\pm 1$ oder $\pm 2$, stärker bevorzugt $\pm 1$.

Des Weiteren ist es erfindungsgemäß bevorzugt, wenn eine der Emitterverbindungen ein Makromolekül (Polymer, Oligomer, Dendrimer), wie z.B. ein Dendrimer oder ein sternförmiges Molekül bildet.

Kleine Moleküle im Sinne der vorliegenden Erfindung sind keine Polymere, Oligomere, Dendrimere oder Mischungen daraus (Blends). Insbesondere sind mehrfache Wiederholungseinheiten, wie sie typischerweise in Polymeren auftreten, nicht in kleinen Molekülen zu finden. Das Molekulargewicht kleiner Moleküle liegt typischerweise unterhalb des Molekulargewichts von Polymeren und im Bereich von Oligomeren mit einer niedrigen Anzahl von Wiederholungseinheiten und darunter.

Das Molekulargewicht kleiner Moleküle ist bevorzugt kleiner oder gleich 4000 g/mol, ganz bevorzugt kleiner oder gleich 3000 g/mol und ganz besonders bevorzugt kleiner oder gleich 2000 /mol.

Polymere oder Makromoleküle im Sinne der vorliegenden Erfindung haben 10 bis 10000, bevorzugt 20 bis 5000 und ganz bevorzugt 50 bis 2000 Wiederholungseinheiten. Oligomere im Sinne der vorliegenden Erfindung haben bevorzugt 2 bis 9 Wiederholungseinheiten .

Der Grad der Verzweigung der Polymere und Oligomere liegt zwischen 0 (lineares Polymer ohne Verzweigung) und 1 (vollständig verzweigtes Dendrimer). Der hierin verwendete Begriff Dendrimer entspricht der Definition von M. Fischer et al. in Angew. Chem., Int. Ed. 1999, 38, 885).

Das Molekulargewicht (MW) von erfindungsgemäßen Polymeren liegt bevorzugt im Bereich von 10000 bis 2000000 g/mol, ganz bevorzugt zwischen 100000 und 1500000 g/mol und ganz besonders bevorzugt zwischen 200000 und 1000000 g/mol. Die Bestimmung von MW kann mittels Verfahren durchgeführt werden, die dem Fachmann auf dem Gebiet sehr gut bekannt sind, nämlich mittels GPC (Gel Permeation Chromatography) mit Polystyrrol als interner Standard.

Ein Blend ist eine Mischung enthaltend wenigstens eine polymere, dendrimere oder oligomere Komponente.

Beispiele für erfindungsgemäße Salze bzw. Systeme sind der folgenden Übersicht (Formeln (191) bis (195)) zu entnehmen

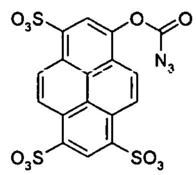

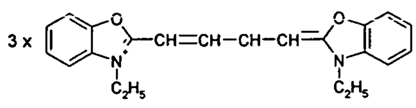

Formel (191)

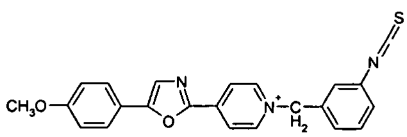

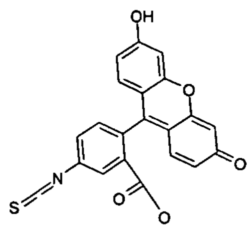

Formel (192)

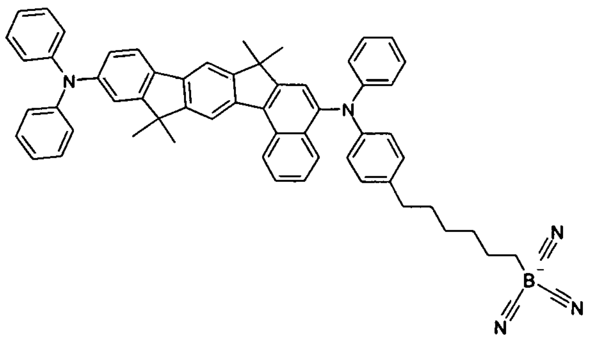

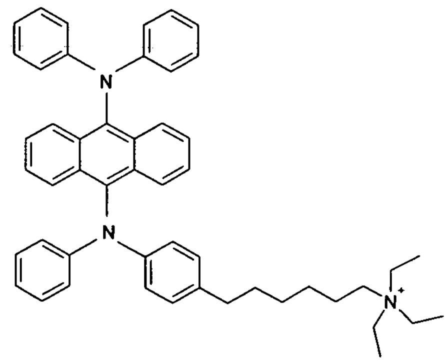

Formel (193)

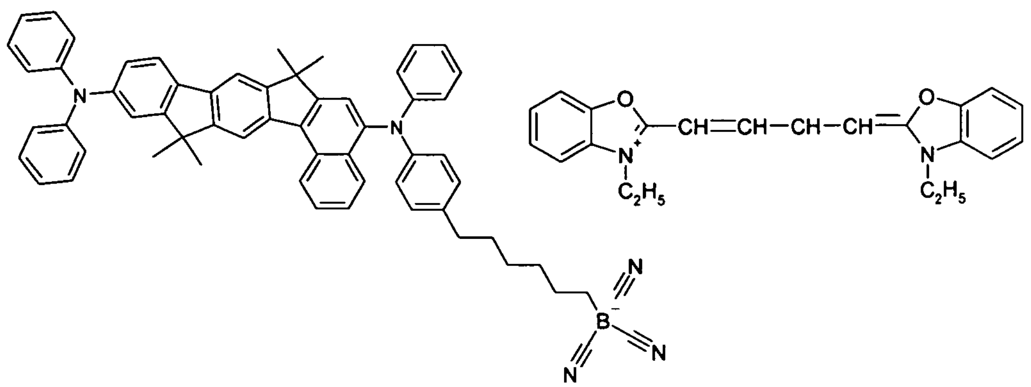

Formel (194)

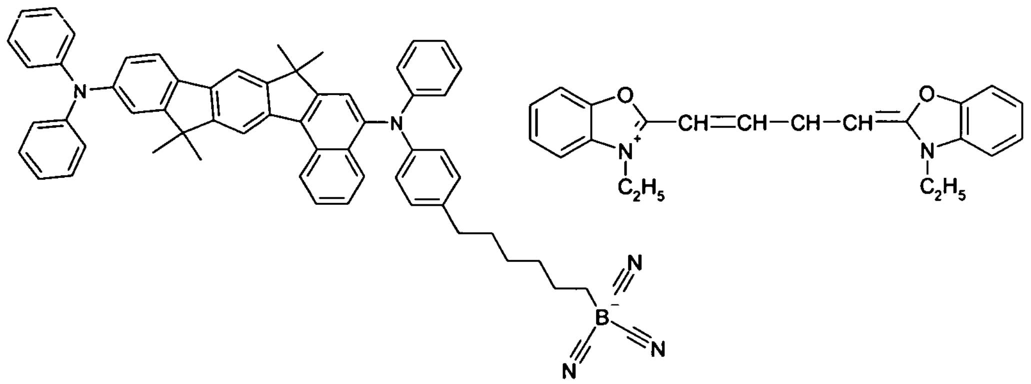

Formel (195)

Weitere Beispiele für erfindungsgemäße Salze kann man aus Kombinationen von Verbindungen der Formeln (1) bis (48), und Verbindungen der Formeln (89) bis (98) oder von Verbindungen der Formeln (1) bis (48), Verbindungen der Formeln (89) bis (98) und Verbindungen der Formeln (191) bis (195) bilden.

Die Emissionsbande einer Emitterverbindung in dem erfindungsgemäßen Salz bzw. System liegt vorzugsweise in einem Wellenlängenbereich, der mit dem Wellenlängenbereich der Absorptionsbande einer weiteren Emitterverbindung überlappt.

Das Maximum der Emissionsbande von $[E_1]^p$ liegt vorzugsweise im Wellenlängenbereich von blauem Licht, und die Maxima der Emissionsbanden von $[E_2]^k$ und $[E_3]^k$ liegen vorzugsweise im Wellenlängenbereich von grünem oder rotem Licht.

Die Emissionsbande von $[E_1]^p$ liegt vorzugsweise in einem Wellenlängenbereich, der mit den Wellenlängenbereichen der Absorptionsbanden von $[E_2]^k$ und $[E_3]^k$ teilweise überlappt.

Die vorliegende Erfindung betrifft auch ein Verfahren zur Herstellung eines erfindungsgemäßen Salzes bzw.

Systems umfassend die folgenden Schritte:

(a) Bereitstellen einer Mehrzahl von Salzen, wobei jedes Salz nur eine Emitterverbindung als Kation oder Anion aufweist und wobei mindestens eine Emitterverbindung eine positive Ladung und mindestens eine Emitterverbindung eine negative Ladung aufweist;

(b) jeweiliges Lösen der bereitgestellten Salze in einem polaren Lösungsmittel, so dass entsprechend der Anzahl der Salze eine Mehrzahl von Lösungen vorliegt;

(c) Mischen der Lösungen;

(d) Kristallisieren eines Salzes, das die mindestens eine Emitterverbindung mit der positiven Ladung als ein Kation und die mindestens eine Emitterverbindung mit der negativen Ladung als ein Anion enthält.

Der oben genannte Schritt (a) des erfindungsgemäßen Verfahrens ist so zu verstehen, dass jedes bereitgestellte Salz nur entweder eine Emitterverbindung als Kation oder als Anion hat und das jeweilige Gegenion dazu keine Emitterverbindung darstellt. Als kationische Gegenionen kommen hier Alkalimetall- oder Erdalkalimetall-Ionen in Betracht. Als anionische Gegenionen werden hier Halogenide bevorzugt.

Unter dem Begriff "jeweiliges Lösen" in Schritt (b) versteht man, dass genauso viele Lösungen hergestellt werden, wie Salze in Schritt (a) bereitgestellt werden. Bevorzugt werden hier polare Lösungsmittel, wie Wasser, Alkohole, wie Methanol, Ethanol, iso-Propanol, etc, Ketone, wie Aceton, 2-Butanon, Acetylaceton, etc., Esther, wie Ethylacetat, Benzoesäureester, etc., cyclische Ether, wie THF und Dioxan, Amide, wie DMF, DMAC, NMP, Sulfoxide wie DMSO, Sulfone, wie Slufolan, etc verwendet. Wasser ist als Lösungsmittel besonders bevorzugt. Die Lösungen haben vorzugsweise eine Molarität im Bereich von 0.001 mol/l bis 10 mol/l, bevorzugt 0.01 mol/l bis 1 mol/l, besonders bevorzugt 0.05 mol/l bis 0.5 mol/l bezogen auf das jeweils eingesetzte Emittersalz.

Nach dem jeweiligen Lösen der Salze werden die Lösungen miteinander vermengt, indem sie ineinander gegossen werden. Es ist hier in der Regel nicht von Bedeutung, welche Lösung auf welche gegeben wird. Es werden vorzugsweise solche Salze verwendet, bei denen die jeweiligen Gegenionen miteinander ein in dem entsprechenden Lösungsmittel leicht lösliches Salz bildet. Vorzugsweise kristallisiert ein Salz aus, welches als Kationen und Anionen Emitterverbindungen enthält, während die Gegenionen der eingesetzten Salze in Lösung bleiben. Zum weiteren Reinigen der Salze können eine oder mehrere weitere Umkristallisationen vorgenommen werden.

In einer besonders bevorzugten Ausführungsform des erfindungsgemäßen Verfahrens werden in Schritt (a) zwei Salze $[E_1]^+Y^-$ und $[E_2]^-X^+$ bereitgestellt, wobei $[E_1]^+$ und $[E_2]^-$ Emitterverbindungen und $Y^-$ und $X^+$ entsprechende Gegenionen darstellen. Auf diese Weise wird in Schritt (d) ein Salz $[E_1]^+[E_2]^-$ erhalten. Die Gegenionen $Y^-$ und $X^+$ bilden vorzugsweise gemeinsam ein Salz $X^+Y^-$, das leichter löslich ist als $[E_1]^+[E_2]^-$ und somit im entsprechenden Lösungsmittel vorzugsweise in Lösung bleibt während $[E_1]^+[E_2]^-$ ausfällt oder auskristallisiert.

In einer weiteren bevorzugten Ausführungsform des erfindungsgemäßen Verfahrens werden in Schritt (a) drei Salze $[E_1]^{2+}[2Y]^-$, $[E_2]^-[X]^+$ und $[E_3]^-$ $[X]^+$ bereitgestellt, wobei $[E_3]^-$, $[E_1]^{2+}$ und $[E_2]^-$ Emitterverbindungen und $Y^-$ und $X^+$ entsprechende Gegenionen darstellen, wobei jedoch $X^+$ bei jedem Auftreten gleich oder verschieden voneinander sein kann, und es auch erfindungsgemäß abgedeckt ist, dass statt zwei Gegenionen $Y^-$ auch ein zweifach negatives Gegenion vorliegen kann. Auf die entsprechende Weise wird in Schritt (d) ein Salz $[E_3]^-[E_1]^{2+}[E_2]^-$ erhalten. Die Gegenionen $Y^-$ und $X^+$ bilden auch hier gemeinsam ein Salz $X^+Y^-$, welches leichter löslich ist als $[E_3]^-[E_1]^{2+}[E_2]^-$.

Die Anionen $Y^-$ können dabei die Folgenden sein: F-, Cl-, Br-, I-, etc., zusammengesetzte Anionen wie OH-, CN-, SCN-, N3-, $BF_4^-$, $PF_6^-$, $PO_4^{3-}$, $SO_4^{3-}$, etc., organische Anionen wie Carboxylate, Alkoholoate, Thiolate, Sulfonate, etc.

Die Kationen $X^+$ können dabei die Folgenden sein: $H^+$, $Li^+$, $Na^+$, $K^+$, $Cs^+$, $Mg^{2+}$, $Ca^{2+}$ etc., zusammengesetzte Kationen wie $NH_4^+$, $PH_4^+$, etc., organische Kationen wie Ammonium, Phosphonium, etc.

Unter einem "$C_{1-40}$-Alkyl" werden in der vorliegenden Erfindung vorzugsweise lineare, verzweigte oder cyclische Alkylgruppen verstanden. Die linearen Alkylgruppen haben vorzugsweise 1 bis 6, 1 bis 10 oder 1 bis 40 Kohlenstoffatome. Die verzweigten oder cyclischen Alkylgruppen haben vorzugsweise 3 bis 6, 3 bis 10 oder 3 bis 40 Kohlenstoffatome. Bevorzugt sind Alkylgruppen mit 1 bis 6, bzw. 3 bis 6 Kohlenstoffatomen, besonders bevorzugt 1 bis 3, bzw. 3 Kohlenstoffatomen. Ein oder mehrere Wasserstoffatome an diesen Alkylgruppen können durch ein Fluoratom ersetzt sein. Außerdem können ein oder mehrere der $CH_2$-Gruppen dieser Einheiten durch NR, O oder S ersetzt sein (R ist dabei ein Rest, der aus der Gruppe ausgewählt ist, die aus H und $C_{1-6}$-Alkyl besteht). Wenn eine oder mehrere der $CH_2$-Gruppen durch NR, O oder S ersetzt ist, ist es besonders bevorzugt, dass nur eine dieser Gruppen ersetzt ist; besonders bevorzugt durch ein O-Atom. Beispiele solcher Verbindungen schließen die folgenden ein: Methyl, Ethyl, n-Propyl, i-Propyl, n-Butyl, i-Butyl, s-Butyl, t-Butyl, 2-Methylbutyl, n-Pentyl, s-Pentyl, n-Hexyl, n-Heptyl, n-Octyl, 2-Ethylhexyl, Trifluormethyl, Pentafluorethyl, 2,2,2-Trifluorethyl, Cyclopentyl, Cyclohexyl, Cycloheptyl und Cyclooctyl. Ebenso versteht man unter $C_{1-4}$-Alkyl, $C_{1-10}$-Alkyl und $C_{1-20}$-Alkyl Alkylgruppen wie vorstehend definiert, mit der Maßgabe, dass sie entsprechend

weniger Kohlenstoffatome enthalten.

Ein mono- oder polycyclischer aromatischer oder heteroaromatischer Kohlenwasserstoffrest enthält vorzugsweise 5 bis 40, stärker bevorzugt 5 bis 20, am bevorzugtesten 5 oder 6 aromatische Ringatome. Ist die Einheit eine aromatische Einheit, so enthält sie vorzugsweise 6 bis 40, stärker bevorzugt 6 bis 20, am bevorzugtesten 6 Kohlenstoffatome als Ringatome. Ist die Einheit eine heteroaromatische Einheit enthält sie 5 bis 40, stärker bevorzugt 5 bis 10, am bevorzugtesten 5 aromatische Ringatome, von denen mindestens eines ein Heteroatom ist. Die Heteroatome sind bevorzugt ausgewählt aus N, O und/oder S. Dabei wird unter einer aromatischen bzw. heteroaromatischen Einheit entweder ein einfacher aromatischer Cyclus, also Benzol, bzw. ein einfacher heteroaromatischer Cyclus, beispielsweise Pyridin, Pyrimidin; Thiophen, etc., oder eine kondensierte Aryl- oder Heteroarylgruppe, beispielsweise Naphthalin, Anthracen, Phenanthren, Chinolin, Isochinolin, Benzothiophen, Benzofuran und Indol etc., verstanden.

Erfindungsgemäße Beispiele für die aromatischen oder heteroaromatischen Kohlenwasserstoffreste sind demgemäß: Benzol, Naphthalin, Anthracen, Phenanthren, Pyren, Chrysen, Benzanthracen, Perylen, Naphthacen, Pentacen, Benzpyren, Furan, Benzofuran, Isobenzofuran, Dibenzofuran, Thiophen, Benzothiophen, Isobenzothiophen, Dibenzothiophen, Pyrrol, Indol, Isoindol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Unter einem mono- oder polycylischen aromatischen Ringsystem versteht man im Sinne dieser Erfindung vorzugsweise ein aromatisches Ringsystem mit 6 bis 60 Kohlenstoffatomen, bevorzugt 6 bis 30, besonders bevorzugt 6 bis 10 Kohlenstoffatomen. Unter einem aromatischen Ringsystem im Sinne der vorliegenden Erfindung soll ein System verstanden werden, das nicht notwendigerweise nur aromatische Gruppen enthält, sondern in dem auch mehrere aromatische durch eine kurze nicht-aromatische Einheit (kleiner 10 % der von H verschiedenen Atome, vorzugsweise kleiner 5 % der von H verschiedenen Atome), wie beispielsweise $sp^3$-hybridisierter C, O, N, etc., unterbrochen sein können. Diese aromatischen Ringsysteme können monocyclisch oder polycyclisch sein, d.h. sie können einen Ring (z.B. Phenyl) oder zwei oder mehr Ringe aufweisen, welche auch kondensiert (z.B. Naphthyl) oder kovalent verknüpft sein können (z.B. Biphenyl), oder eine Kombination von kondensierten und verknüpften Ringen beinhalten.

Bevorzugte aromatische Ringsysteme sind z.B. Phenyl, Biphenyl, Triphenyl, Naphthyl, Anthracyl, Binaphthyl, Phenanthryl, Dihydrophenanthryl, Pyren, Dihydropyren, Chrysen, Perylen, Tetracen, Pentacen, Benzpyren, Fluoren und Inden.

Unter einem mono- oder polycylischen heteroaromatischen Ringsystem versteht man im Sinne dieser Erfindung vorzugsweise ein heteroaromatisches Ringsystem mit 5 bis 60 Ringatomen, bevorzugt 5 bis 30, besonders bevorzugt 5 bis 14 Ringatomen. Das heteroaromatische Ringsystem enthält mindestens ein Heteroatom ausgewählt aus N, O und S (verbleibenden Atome sind Kohlenstoff). Unter einem heteroaromatischen Ringsystem soll zudem ein System verstanden werden, das nicht notwendigerweise nur aromatische oder heteroaromatische Gruppen enthält, sondern in dem auch mehrere aromatische bzw. heteroaromatische Gruppen durch eine kurze nicht-aromatische Einheit (< 10 % der von H verschiedenen Atome, vorzugsweise < 5 % der von H verschiedenen Atome), wie beispielsweise $sp^3$-hybridisierter C, O, N, etc., unterbrochen sein können. Diese heteroaromatischen Ringsysteme können monocyclisch oder polycyclisch sein, d.h. sie können einen Ring (z.B. Pyridyl) oder zwei oder mehr Ringe aufweisen, welche auch kondensiert oder kovalent verknüpft sein können, oder eine Kombination von kondensierten und verknüpften Ringen beinhalten.

Bevorzugte heteroaromatische Ringsysteme sind z.B. 5-gliedrige Ringe wie Pyrrol, Pyrazol, Imidazol, 1,2,3-Triazol, 1,2,4-Triazol, Tetrazol, Furan, Thiophen, Selenophen, Oxazol, Isoxazol, 1,2-Thiazol, 1,3-Thiazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 6-gliedrige Ringe wie Pyridin, Pyridazin, Pyrimidin, Pyrazin, 1,3,5-Triazin, 1,2,4-Triazin, 1,2,3-Triazin, 1,2,4,5-Tetrazin, 1,2,3,4-Tetrazin, 1,2,3,5-Tetrazin, oder kondensierte Gruppen wie Indol, Isoindol, Indolizin, Indazol, Benzimidazol, Benzotriazol, Purin, Naphthimidazol, Phenanthrimidazol, Pyridimidazol, Pyrazinimidazol, Chinoxalinimidazol, Benzoxazol, Naphthoxazol, Anthroxazol, Phenanthroxazol, Isoxazol, Benzothiazol, Benzofuran, Isobenzofuran, Dibenzofuran, Chinolin, Isochinolin, Pteridin, Benzo-5,6-chinolin, Benzo-6,7-chinolin, Benzo-7,8-chinolin, Benzoisochinolin, Acridin, Phenothiazin, Phenoxazin, Benzopyridazin, Benzopyrimidin, Chinoxalin, Phenazin, Naphthyridin, Azacarbazol, Benzocarbolin, Phenanthridin, Phenanthrolin, Thieno[2,3b]thiophen, Thieno[3,2b]thiophen, Dithienothiophen, Isobenzothiophen, Dibenzo-thiophen, Benzothiadiazothiophen oder Kombinationen dieser Gruppen. Besonders bevorzugt sind Imidazol, Benzimidazol und Pyridin.

Unter dem Begriff "Aryl" oder "Arylgruppe" versteht man ein mono- oder polycylisches aromatisches oder heteroaromatisches Ringsystem, wie vorstehend definiert.

Unter dem Begriff "Alkylsilyl" versteht man Mono-($C_{1-12}$-alkyl)-silylgruppen, Di-($C_{1-12}$-alkyl)-silylgruppen und Tri-($C_{1-12}$-alkyl)-silylgruppen.

Unter einer "Mono-($C_{1-12}$-alkyl)-silylgruppe" versteht man in der vorliegenden Erfindung eine ($SiH_2$)-Gruppe, die mit einer linearen oder verzweigten Alkylgruppe (wie oben definiert) mit 1 bzw. 3 bis 12 Kohlenstoffatomen, stärker bevorzugt 1 bzw. 3 bis 6 Kohlenstoffatomen verknüpft ist. Unter einer "Di-($C_{1-12}$-alkyl)-silylgruppe" versteht man in der vorliegenden Erfindung eine (SiH)-Einheit, die mit zwei bei jedem Auftreten gleich oder verschiedenen linearen oder verzweigten Alkylgruppen (wie oben definiert) mit 1 bzw. 3 bis 12 Kohlenstoffatomen, stärker bevorzugt 1 bzw. 3 bis 6 Kohlenstoffatomen verknüpft ist. Unter einer "Tri-($C_{1-12}$-alkyl)-silylgruppe" versteht man in der vorliegenden Erfindung eine (Si)-Einheit, die mit drei bei jedem Auftreten gleich oder verschiedenen linearen oder verzweigten Alkylgruppen (wie oben definiert) mit 1 bzw. 3 bis 12 Kohlenstoffatomen, stärker bevorzugt 1 bzw. 3 bis 6 Kohlenstoffatomen verknüpft ist. Die oben in Verbindung mit "$C_{1-40}$-Alkyl" angegeben Beispiele gelten auch für die hier vorhandenen Alkylgruppen, sofern sie die entsprechende Anzahl an Kohlenstoffatomen aufweisen.

Unter "Silyl" versteht man in der vorliegenden Verbindung eine Silylgruppe mit 1 bzw. 3 bis 5 Siliciumatomen, die linear oder verzweigt ist. Beispiele hierfür sind Monosilyl, Disilyl, Trisilyl, Tetrasilyl und Pentasilyl.

Unter "Arylsilyl" versteht man in der vorliegenden Erfindung eine $Si_1$-Silylgruppe, die mit einem, zwei oder drei mono- oder polycyclischen aromatischen oder heteroaromatischen Ringsystemen mit 5 bis 60 aromatischen Ringatomen substituiert ist.

Unter "Alkoxyalkyl" versteht man in der vorliegenden Erfindung eine monovalente Ethereinheit mit zwei linearen oder verzweigten Alkylgruppen mit 1 bzw. 3 bis 12, stärker bevorzugt 1 bzw. 3 bis 6 Kohlenstoffatomen, die über ein Sauerstoffatom gebunden sind. Die oben in Verbindung mit der Definition von "$C_{1-40}$-Alkyl" angegebenen Beispiele gelten auch hierfür die vorhandenen Alkylgruppen, sofern sie die entsprechende Atomanzahl aufweisen.

Unter "Arylalkoxyalkyl" versteht man in der vorliegenden Erfindung eine monovalente Einheit wie vorstehend für "Alkoxyalkyl" definiert, wobei eine Alkylgruppe mit einem Aryl substituiert ist, das ein mono- oder polycyclisches aromatisches oder heteroaromatisches Ringsystem mit 5 bis 60 aromatischen Ringatomen wie vorstehend definiert darstellt.

Unter "Alkylthioalkyl" versteht man in der vorliegenden Erfindung eine monovalente Thioethereinheit mit zwei linearen oder verzweigten Alkylgruppen mit 1 bzw. 3 bis 12, stärker bevorzugt 1 bzw. 3 bis 6 Kohlenstoffatomen, die über ein Schwefelatom gebunden sind. Die oben in Verbindung mit der Definition von "$C_{1-40}$-Alkyl" angegebenen Beispiele gelten auch hier für die vorhandenen Alkylgruppen, sofern sie die entsprechende Atomanzahl aufweisen.

Unter "Alkylsulfon" wird in der vorliegenden Erfindung eine Einheit $S(=O)_2$- verstanden, die mit einer Alkylgruppe mit 1 bis 12 Kohlenstoffatomen substituiert ist. Die oben in Verbindung mit der Definition von "$C_{1-40}$-Alkyl" angegebenen Beispiele gelten auch hier für die vorhandenen Alkylgruppen, sofern sie die entsprechende Atomanzahl aufweisen.

Unter "$C_{1-12}$-Alkylsulfoxid" wird in der vorliegenden Erfindung eine Einheit -S(=O)-verstanden, die mit einer Alkylgruppe mit 1 bis 12 Kohlenstoffatomen substituiert ist. Die oben in Verbindung mit der Definition von "$C_{1-40}$-Alkyl" angegebenen Beispiele gelten auch hier für die vorhandenen Alkylgruppen, sofern sie die entsprechende Atomanzahl aufweisen.

Ein weiterer Gegenstand der vorliegenden Erfindung ist eine Multilayer-Struktur, die eine Schicht, die eine erfindungsgemäßes Salz oder System enthält, umfasst.

Unter eine Multilayer-Struktur versteht man in der vorliegenden Erfindung eine Mehrschicht-Struktur aus zwei oder mehr Schichten, die nacheinander vorzugsweise auf einen Glasträger aufgebracht werden. Die Schichten können einzelne erfindungsgemäße Verbindungen enthalten. Es ist bevorzugt, dass die Schichten weitere Verbindungen, Polymere oder Oligomere mit unterschiedlichen Eigenschaften umfassen.

(Ein weiterer Gegenstand der vorliegenden Erfindung ist eine Formulierung, insbesondere eine Lösung, Dispersion oder Emulsion, enthaltend mindestens eine erfindungsgemäßes Salz oder System und mindestens ein Lösungsmittel. Als Lösungsmittel können alle denkbaren eingesetzt werden, die in der Lage sind die erfindungsgemäßen Salze bzw. Systeme zu lösen oder mit ihnen eine Suspension zu bilden. Folgende Lösungsmittel sind hierbei erfindungsgemäß bevorzugt: Dichlormethan, Trichlormethan, Monochlorbenzol, o-Dichlorbenzol, Tetrahydrofuran, Anisol, Morpholin, Toluol, o-Xylol, m-Xylol, p-Xylol, 1,4-Dioxan, Aceton, Methylethylketon, 1,2-Dichlorethan, 1,1,1-Trichlorethan, 1,1,2,2-Tetrachlorethan, Ethylacetat, n-Butylacetat, Dimethylformamid, Dimethylacetamid, Dimethylsulfoxid, Tetralin, Decalin, Indan und/oder Mischungen davon.

Die Konzentration des erfindungsgemäßen Salzes in der Lösung ist vorzugsweise 0,1 bis 10 Gew.-%, stärker bevorzugt 0,5 bis 5 Gew.-%, bezogen auf das Gesamtgewicht der Lösung. Optional umfasst die Lösung auch einen oder mehrere Bindemittel um die rheologischen Eigenschaften der Lösung entsprechend einzustellen, wie beispielsweise in der WO 2005/055248 A1 beschrieben.

Nach dem angemessenen Vermischen und Altern der Lösungen werden diese in eine der folgenden Kategorien eingeteilt: "Vollständige" Lösung, "grenzwertige" Lösung oder unlöslich. Die Grenzlinie zwischen diesen Kategorien wird anhand der Löslichkeitsparameter gezogen. Die entsprechenden Werte können aus der Literatur entnommen werden, wie. beispielsweise aus "Crowley, J. D., Teague, G. S. Jr. und Lowe, J. W. Jr., Journal of Paint Technology, 38, No.

496, 296 (1966)".

Lösungsmittelmischungen können auch verwendet werden und werden so identifiziert, wie in "Solvents, W.H. Ellis, Federation of Societies for Coatings Technology, S. 9 bis 10, 1986" beschrieben werden. Solche Verfahren können zu einer Mischung von sogenannten "nicht"-Lösungsmitteln führen, die die Zusammensetzung lösen, obwohl es wünschenswert ist, wenigstens ein wirkliches Lösungsmittel in der Mischung zu haben.

Eine weitere bevorzugte Form der Formulierung ist eine Emulsion, und stärker bevorzugt eine Miniemulsion, die insbesondere als Heterophasensysteme hergestellt werden, in denen stabile Nanotröpfchen einer ersten Phase in einer zweiten kontinuierlichen Phase dispergiert werden.

Sowohl eine Miniemulsion, worin die kontinuierliche Phase eine polare Phase ist, als auch eine inverse Miniemulsion, worin die kontinuierliche Phase eine nicht polare Phase ist, können in der vorliegenden Erfindung verwendet werden. Die bevorzugte Form ist eine Miniemulsion. Um die kinetische Stabilität der Emulsion zu erhöhen, können auch Tenside beigemischt werden. Die Wahl der Lösungsmittel für zweiphasige Systeme, der Tenside und der Verarbeitung zu einer stabilen Miniemulsion sollten für einen Fachmann auf diesem Gebiet auf Basis seinen Fachwissens oder durch zahlreiche Veröffentlichungen bekannt sein, wie zum Beispiel ein umfassender Artikel von Landfester in Annu. Rev, Mater. Res. (06), 36, S.231.

Zur Verwendung von sogenannten Dünnschichten in elektronischen oder optoelektronischen Vorrichtungen kann das erfindungsgemäße Salz oder eine Formulierung dessen durch ein entsprechend geeignetes Verfahren abgeschieden werden. Flüssigbeschichtung von Vorrichtungen, wie zum Beispiel von OLEDs, ist wünschenswerter als Vakuumabscheidungstechniken. Abscheidungsverfahren aus Lösung sind besonders bevorzugt. Bevorzugte Abscheidungstechniken schließen, ohne die Erfindung entsprechend einzuschränken, Tauchbeschichtung, Spin-coating, Tintenstrahl-Druck, "Letter-press"-Druck, "Screenprinting", "Doctor Blaid"-Beschichten, "Rollerprinting", "Reverse-Rollerprinting", Offset-Lithographie, flexographisches Drucken, "Webprinting", Sprühbeschichten, Pinselbeschichten oder "Padprinting" und "Slot-die coating" ein. Tintenstrahldruck ist besonders bevorzugt und es ermöglicht die Herstellung von hochauflösenden Displays.

Die erfindungsgemäßen Lösungen können auf vorgefertigte Vorrichtungssubstrate mithilfe von Tintenstrahldruck oder durch Mikroverabreichung aufgebracht werden. Bevorzugt werden hierzu industrielle piezoelektrische Druckköpfe, wie von Aprion, Hitachie-Koki, Inkjet Technology, On Target Technology, Picojet, Spectra, Trident, Xaar verwendet werden, um die organischen Halbleiterschichten auf ein Substrat aufzubringen. Zusätzlich können auch halbindustrielle Druckköpfe, wie solche von Brother, Epson, Konika, Seiko Instruments, Toshiba TEC oder eindüsige Mikroverabreichungsgeräten, wie sie zum Beispiel von Mikrodrop und Mikrofab hergestellt werden, verwendet werden.

Damit das erfindungsgemäße Salz durch Tintenstrahl-Druck oder Mikroverabreichung aufgebracht werden kann, sollte sie zunächst in einem geeigneten Lösungsmittel gelöst werden. Die Lösungsmittel müssen die oben genannten Anforderungen erfüllen und dürfen nicht irgendwelche nachteiligen Wirkungen auf den ausgewählten Druckkopf haben. Zusätzlich sollten die Lösungsmittel einen Siedepunkt von über 100°C, vorzugsweise über 140°C und stärker bevorzugt über 150°C haben, um Verarbeitungsprobleme zu vermeiden, die durch das Austrocken der Lösung im Inneren des Druckkopfes hervorgerufen werden. Neben den oben genannten Lösungsmitteln sind auch die folgenden Lösungsmittel geeignet: Substituierte und nicht substituierte Xylolderivate, Di-$C_{1}$-$_{2}$- Alkylformamide, substituierte und nicht substituierte Anisole und andere Phenoletherderivate, substituierte Heterozyklen, wie substituierte Pyridine, Pyrapsine, Pyrimidine, Pyrrolidinone, substituierte und nicht substituierte N, N-Di-$C_{1-2}$-Alkylaniline und andere fluorierte oder chlorierte Aromaten.

Ein bevorzugtes Lösungsmittel für die Ablagerung des erfindungsgemäßen Salzes durch Tintenstrahl-Druck umfasst ein Benzolderivat, das einen durch einen oder mehrere Substituenten substituierten Benzolring aufweist, worin die Gesamtzahl der Kohlenstoffatome des einen oder der mehreren Substituenten wenigstens drei ist. So kann zum Beispiel das Benzolderivat mit einer Propylgruppe oder drei Methylgruppen substituiert sein, wobei in jedem Fall die Gesamtanzahl der Kohlenstoffatome wenigstens drei sein muss. Ein solches Lösungsmittel ermöglicht die Bildung einer Tintenstrahl-Flüssigkeit, die das Lösungsmittel mit dem erfindungsgemäßen Salz umfasst, und vermindert oder verhindert das Verkleben der Düsen und die Trennung der Komponenten während dem Aufsprühen. Das/die Lösungsmittel kann/können (ein) solche(s) sein, das/die aus der folgenden Beispielsliste ausgewählt wird/werden: Dodecylbenzol, 1-Methyl-4-tert-butylbenzol, Terpineollimonen, Isodurol, Terpinolen, Cymol und Dethylbenzol. Das Lösungsmittel kann auch eine Lösungsmittelmischung aus zwei oder mehr Lösungsmittel sein, wobei jedes der Lösungsmittel vorzugsweise einen Siedepunkt von größer 100 °C, stärker bevorzugt größer 140 °C aufweist. Solche Lösungsmittel fördern die Filmbildung der abgelagerten Schicht und vermindern Schichtfehler.

Die Tintenstrahl-Flüssigkeit, (das heißt eine Mischung vorzugsweise aus Lösungsmittel(n), Bindemittel und der erfindungsgemäßen Verbindung) weist vorzugsweise eine Viskosität bei 20°C von 1 bis 100 mPa·s, stärker bevorzugt 1 bis 50 mPa·s und am stärksten bevorzugt 1 bis 30 mPa·s auf.

Die/das erfindungsgemäße Salz oder Formulierung kann zusätzlich ein oder mehrere weitere Komponenten wie zum Beispiel oberflächenaktive Substanzen, Gleitmittel, Benetzungsmittel, Dispergiermittel, wasserabweisende Mittel, Haftmittel, Fließverbesserer, Antischäumungsmittel, Luftabscheidungsmittel, Verdünnungsmittel, die reaktive oder

nicht reaktive Substanzen sein können, Hilfsmittel, Farbmittel, Farbstoffe oder Pigmente, Sensibilisatoren, Stabilisatoren oder Inhibitoren umfassen.

Eine erfindungsgemäße Formulierung kann nicht nur hergestellt werden, indem ein als Feststoff gewonnenes erfindungsgemäßes Salz in einem Lösungsmittel gelöst wird, sondern auch indem die oben genannten Verfahrensschritte (a) bis (c) durchgeführt werden, und anschließend die nicht erwünschten Gegenionen durch chromatographische Verfahren oder Ionenaustauschprozessen aus der Lösung entfernt werden.

Ein weiterer Gegenstand der Erfindung ist die Verwendung der oben genannten erfindungsgemäßen Salze bzw. Systeme in einer organischen Elektrolumineszenzvorrichtung. Die erfindungsgemäßen Salze oder Systeme sind dabei bevorzugt als oder in einer elektrolumineszierende(n) Schicht ausgebildet. Eine Schicht wird vorzugsweise gebildet, indem eine erfindungsgemäße Formulierung auf einen Träger aufgebracht und anschließend das Lösungsmittel entfernt wird.

In einer weiteren bevorzugten Ausführungsform enthält die Formulierung mindestens eine weitere neutrale organische funktionelle Verbindung gewählt aus HTM (hole transport Material), ETM (electron transport material), Host und Emittern.

Ein weiterer Gegenstand der vorliegenden Erfindung ist eine elektronische Vorrichtung enthaltend ein erfindungsgemäßes Salz bzw. System oder Formulierung.

Die elektronische Vorrichtung ist vorzugsweise eine organische Elektrolumineszenzvorrichtung, enthaltend vorzugsweise eine Kathode, eine Anode und mindestens eine organische Schicht, wobei die organische Schicht das erfindungsgemäße Salz bzw. System oder Formulierung enthält.

Wie eben angeführt ist die organische Schicht, die das erfindungsgemäße Salz bzw. System oder Formulierung enthält, vorzugsweise die emittierende Schicht. In einer sehr bevorzugten Ausführungsform enthält die organische Schicht mindestens eine weitere neutrale organische funktionelle Verbindung ausgewählt aus HTM (hole transport Material), ETM (electron tranpsort material), Host und Emittern, besonders bevorzugt mindestens einem Hostmaterial.

Als Hostmaterialien kommen hierfür Materialien verschiedener Stoffklassen in Frage. Bevorzugte Hostmaterialien sind ausgewählt aus den Klassen der Oligoarylene (z. B. 2,2',7,7'-Tetraphenylspirobifluoren gemäß EP 676461 oder Dinaphthylanthracen), insbesondere der Oligoarylene enthaltend kondensierte aromatische Gruppen wie z.B. Anthracen, Benzanthracen, Benzphenanthren (DE 102009005746.3, WO 2009/069566), Phenanthren, Tetracen, Coronen, Chrysen, Fluoren, Spirofluoren, Perylen, Phthaloperylen, Naphthaloperylen, Decacyclen, Rubren, der Oligoarylenvinylene (z. B. DPVBi = 4,4'-Bis(2,2-diphenylethenyl)-1,1'-biphenyl) oder Spiro-DPVBi gemäß EP 676461), der polypodalen Metallkomplexe (z. B. gemäß WO 04/081017), insbesondere Metallkomplexe von 8-Hydroxychinolin, z.B. $AlQ_3$ (= Aluminium(III)tris(8-hydroxychinolin)) oder Bis(2-methyl-8-chinolinolato)-4-(phenylphenolinolato)aluminium, auch mit Imidazol-Chelat (US 2007/0092753 A1) sowie der Chinolin-Metallkomplexe, Aminochinolin-Metallkomplexe, Benzochinolin-Metallkomplexe, der lochleitenden Verbindungen (z. B. gemäß WO 2004/058911), der elektronenleitenden Verbindungen, insbesondere Ketone, Phosphinoxide, Sulfoxide, etc. (z. B. gemäß WO 2005/084081 und WO 2005/084082), der Atropisomere (z. B. gemäß WO 2006/048268), der Boronsäurederivate (z. B. gemäß WO 2006/117052) oder der Benzanthracene (z. B. gemäß WO 2008/145239).

Besonders bevorzugte Hostmaterialien sind ausgewählt aus den Klassen der Oligoarylene, enthaltend Anthracen, Benzanthracen und/oder Pyren oder Atropisomere dieser Verbindungen. Unter einem Oligoarylen im Sinne dieser Erfindung soll eine Verbindung verstanden werden, in der mindestens drei Aryl- bzw. Arylengruppen aneinander gebunden sind.

Bevorzugte Hostmaterialien sind insbesondere ausgewählt aus Verbindungen der Formel (195a),

$$Ar^4\text{-}(Ar^5)_p\text{-}Ar^6 \qquad \text{Formel (195a)}$$

wobei $Ar^4$, $Ar^5$, $Ar^6$ bei jedem Auftreten gleich oder verschieden eine Aryl- oder Heteroarylgruppe mit 5 bis 30 aromatischen Ringatomen ist, die mit einem oder mehreren Resten $R^1$ substituiert sein kann, und $R^1$ und p dieselbe Bedeutung haben, wie oben beschrieben; dabei gilt, dass die Summe der $\pi$-Elektronen in $Ar^4$, $Ar^5$ und $Ar^6$ mindestens 30 beträgt, wenn p = 1 ist, und mindestens 36 beträgt, wenn p = 2 ist, und mindestens 42 beträgt, wenn p = 3 ist.

Besonders bevorzugt steht in den Hostmaterialien der Formel (195a) die Gruppe $Ar^5$ für Anthracen, welches durch einen oder mehrere Reste $R^1$ substituiert sein kann, und die Gruppen $Ar^4$ und $Ar^6$ sind in 9- und 10-Position gebunden. Ganz besonders bevorzugt ist mindestens eine der Gruppen $Ar^4$ und/oder $Ar^6$ eine kondensierte Arylgruppe, ausgewählt aus 1- oder 2-Naphthyl, 2-, 3- oder 9-Phenanthrenyl oder 2-, 3-, 4-, 5-, 6- oder 7-Benzanthracenyl, welche jeweils durch einen oder mehrere Reste $R^1$ substituiert sein kann. Anthracen-basierte Verbindungen sind beschrieben in US 2007/0092753 A1 und US 2007-0252517 A1, z.B. 2-(4-Methylphenyl)-9,10-di-(2-naphthyl)anthracen, 9-(2-Naphthyl)-10-(1,1'-biphenyl)-anthracen und 9,10-Bis[4-(2,2-diphenylethenyl)phenyl]anthracen, 9,10-Diphenylanthracen, 9,10-Bis(phenylethynyl)anthracen und 1,4-Bis(9'-ethynylanthracenyl)benzol. Bevorzugt sind auch Verbindungen mit zwei Anthraceneinheiten (US 2008/0193796 A1), z.B. 10, 10'-Bis[1,1',4', 1"]terphenyl-2-yl-9,9'-bisanthracenyl.

Weitere bevorzugte Verbindungen sind Derivate von Arylamin, Styrylamin, Fluorescein,

Diphenylbutadien" Tetraphenylbutadien, Cyclopentadiene, Tetraphenylcyclopentadien, Pentaphenylcyclopentadien, Cumarin, Oxadiazol, Bisbenzoxazolin, Oxazol, Pyridin, Pyrazin, Imin, Benzothiazol, Benzoxazol, Benzimidazol (US 2007/0092753 A1), z.B. 2,2',2"-(1,3,5-Phenylen)tris[1-phenyl-1 H-benzimidazol], Aldazin, Stilben, Styrylarylenderivate, z.B. 9,10-Bis[4-(2,2-diphenylethenyl)phenyl]anthracen und Distyrylarylen-Derivate (US 5121029), Diphenylethylen, Vinylanthracen, Diaminocarbazol, Pyran, Thiopyran, Diketopyrrolopyrrol, Polymethin, Zimtsäureestern und Fluoreszenzfarbstoffen.

Besonders bevorzugt sind Derivate von Arylamin und Styrylamin, z.B. TNB (= 4,4'-Bis[N-(1-naphthyl)-N-(2-naphthyl)amino]biphenyl). Metall-Oxinoid-Komplexe wie LiQ oder $AlQ_3$ können als Co-Hosts verwendet werden.

Bevorzugte Verbindung mit Oligoarylen als Matrix:

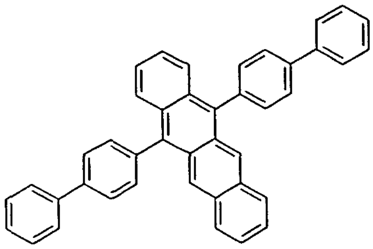

Formel (196)
US 2003/0027016 A1

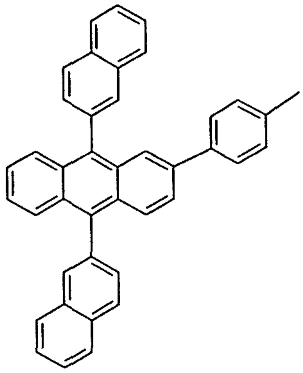

Formel (197)
US 7326371 B2

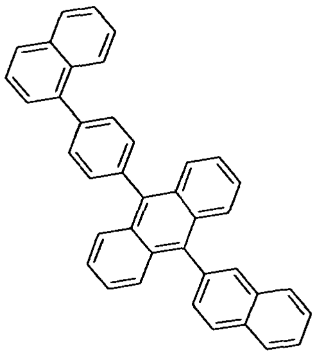

Formel (198)
US 2006/043858 A

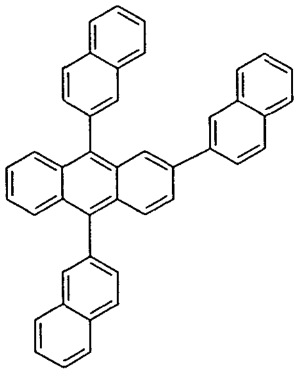

Formel (199)
US 7326371 B2

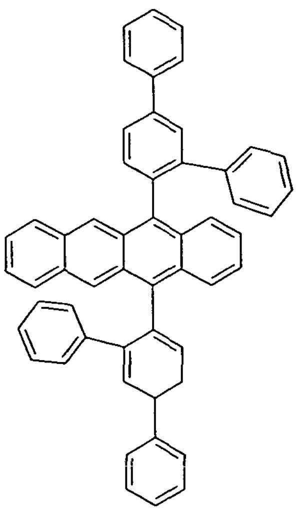

Formel (200)
US 2003/0027016 A1

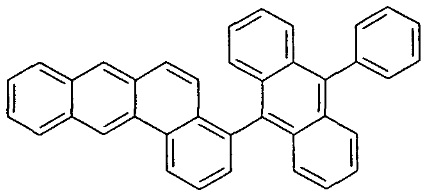

Formel (201)
WO 2008/145239

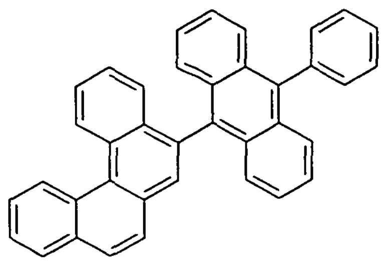

Formel (202)
DE 102009005746.3

Als geeignete Matrixmaterialien in elektronischen Vorrichtungen für das erfindungsgemäße Salz eignen sich bspw. CBP (N,N-Biscarbazolylbiphenyl), Carbazolderivate (z. B. gemäß WO 2005/039246, US 2005/0069729, JP 2004/288381, EP 1205527 oder WO 2008/086851), Azacarbazole (z. B. gemäß EP 1617710, EP 1617711, EP 1731584, JP 2005/347160), Ketone (z. B. gemäß WO 2004/093207 oder gemäß DE 102008033943), Phosphinoxide, Sulfoxide und Sulfone (z. B. gemäß WO 2005/003253), Oligophenylene, aromatische Amine (z. B. gemäß US 2005/0069729), bipolare Matrixmaterialien (z. B. gemäß WO 2007/137725), Silane (z. B. gemäß WO 2005/111172), 9,9-Diarylfluorenderivate (z. B. gemäß DE 102008017591), Azaborole oder Boronester (z. B. gemäß WO 2006/117052), Triazin-Derivate (z. B. gemäß DE 102008036982), Indolocarbazolderivate (z. B. gemäß WO 2007/063754 oder WO 2008/056746), Indenocarbazolderivate (z. B. gemäß der nicht offen gelegten Anmeldung DE 102009023155.2 und DE 102009031021.5), Diazaphospholderivate (z. B. gemäß der nicht offen gelegten Anmeldung DE 102009022858.6), Triazol-Derivate, Oxazole und Oxazol-Derivate, Imidazol-Derivate, Polyarylalkan-Derivate, Pyrazolin-Derivate, Pyrazolon-Derivate, Distyrylpyrazin-Derivate, Thiopyrandioxid-Derivate, Phenylendiamin-Derivate, tertiäre aromatische Amine, Styrylamine, Amino-substituierte Chalcon-Derivate, Indole, Hydrazon-Derivate, Stilben-Derivate, Silazan-Derivate, aromatische Dimethyliden-Verbindungen, Carbodiimid-Derivate, Metallkomplexe von 8-Hydroxychinolin-Derivaten wie z.B. $AlQ_3$, die 8-Hydroxychinolin-Komplexe können auch Triarylaminophenol-Liganden enthalten (US 2007/0134514 A1), Metallkomplex-Polysilan-Verbindungen sowie Thiophen-, Benzothiophenund Dibenzothiophen-Derivate.

Beispiele für bevorzugte Carbazolderivate sind mCP (= 1,3-N,N-dicarbazol-benzol (= 9,9'-(1,3-Phenylen)bis-9H-carbazol), Formel (203), US 2005/0249976), CDBP (= 9,9'-(2,2'-Dimethyl[1,1'-biphenyl]-4,4'-diyl)-bis-9H-carbazol), 1,3-Bis(N,N'-dicarbazol)benzol (= 1,3-Bis(carbazol-9-yl)benzol), PVK (Polyvinylcarbazol), 3,5-Di(9H-carbazol-9-yl)biphenyl sowie die weiteren, unten abgebildeten Verbindungen mit den Formeln (204) bis (207) (s. auch US 2007/0128467, US 2007/0128467).

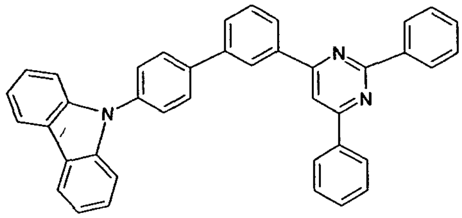

Formel (203)

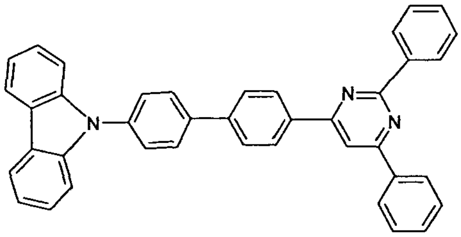

Formel (204)

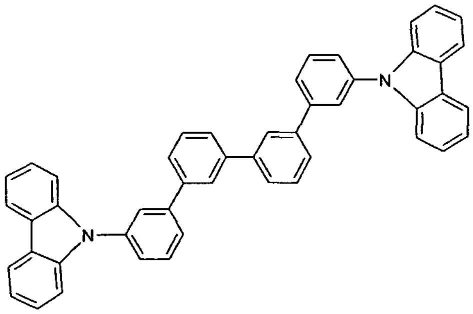

Formel (205)

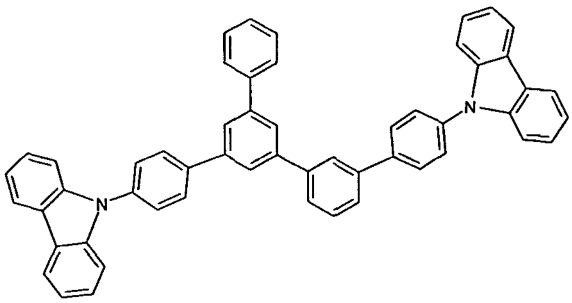

Formel (206)

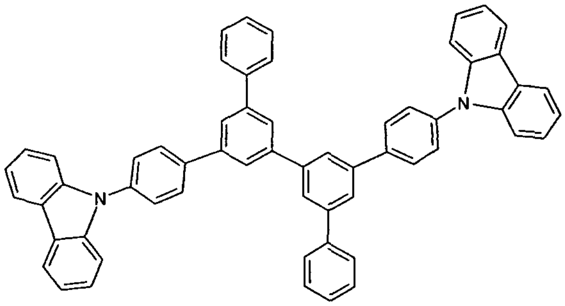

Formel (207)

Weitere bevorzugte Matrixmaterialien im Sinne der vorliegenden Erfindung sind Si-Tetraaryle wie sie bspw. in US 004/209115, US 2004/0209116 US 2007/0087219, US 2007/0087219 und H. Gilman, E.A. Zuech, Chemistry&Industry (London, United Kingdom), 1960, 120 offenbart sind, besonders bevorzugt hierbei sind die Verbindungen der Formeln (208) bis (215).

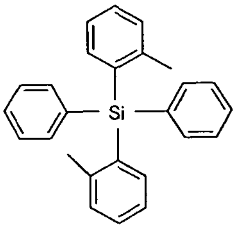

Formel (208)

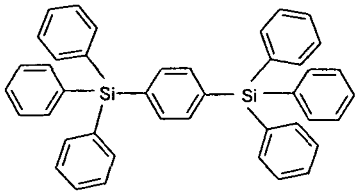

Formel (209)

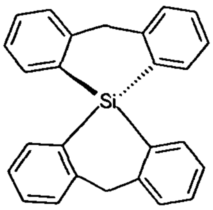

Formel (210)

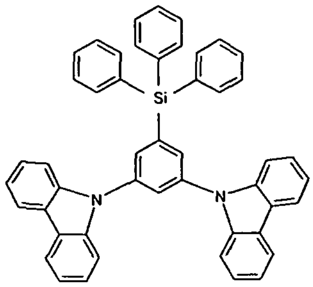

Formel (211)

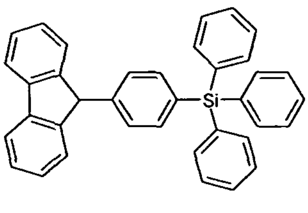

Formel (212)

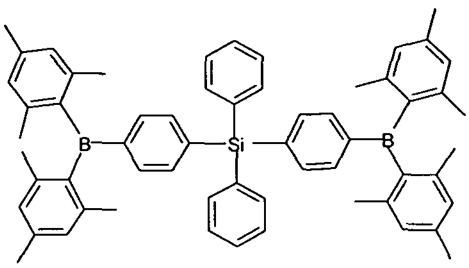

Formel (213)

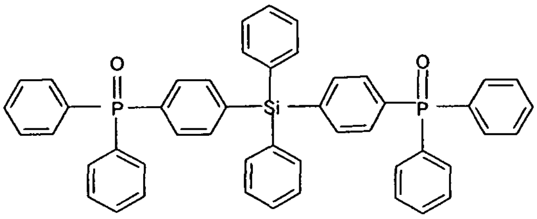

Formel (214)

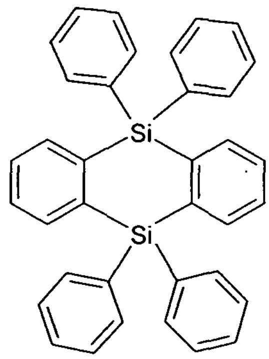

Formel (215)

Besonders bevorzugte Matrixmaterialien für phosphoreszierende Dotanden sind Verbindungen in EP 652273, DE 102009022858.6, DE 102009023155.2, WO 2007/063754 und WO 2008/056746, insbesondere die Verbindungen der Formeln (216) bis (219).

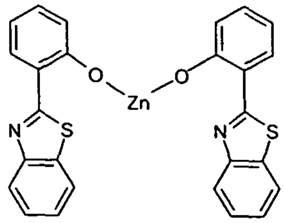

Formel (216)

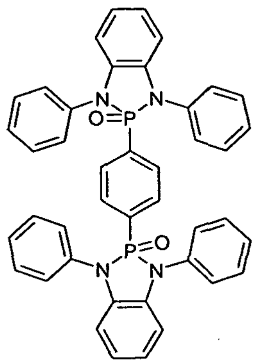

Formel (217)

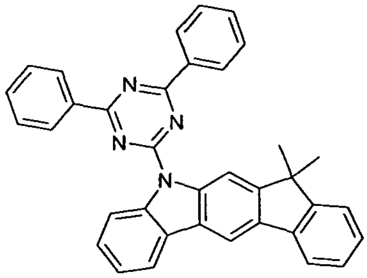

Formel (218)

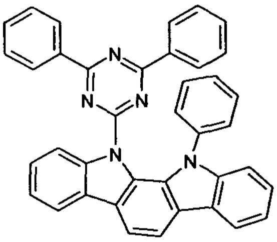

Formel (219)

Die organische Elektrolumineszenzvorrichtung kann darüber hinaus weitere Schichten enthalten, ausgewählt aus jeweils einer oder mehrerer Lochinjektionsschichten, Lochtransportschichten, Lochblockierschichten, Elektronentransportschichten, Elektroneninjektionsschichten, Elektronenblockierschichten, Ladungserzeugungsschichten und/oder Schichten, welche organische oder anorganische P/N-Übergänge erzeugen. Die elektrolumineszierende Vorrichtung kann darüber hinaus weitere emittierende Schichten enthalten. Vorzugsweise sind zwischen zwei emittierenden Schichten sogenannte Interlayer eingebracht, welche beispielsweise eine Excitonen-blockierende Funktion aufweisen. Es sei aber darauf hingewiesen, dass nicht notwendigerweise jede dieser Schichten vorhanden sein muss.

Die organische Elektrolumineszenzvorrichtung, die das erfindungsgemäße Salz bzw. System umfasst, weist vorzugsweise eine planare Form auf und/oder ist Faser-förmig.

Unter einer Faser im Sinne der vorliegenden Erfindung wird jede Form verstanden, in dem das Verhältnis zwischen Länge zu Durchmesser größer oder gleich 10:1, bevorzugt 100:1 ist, wobei es auf die Form des Querschnitts entlang der Längenachse nicht ankommt. Der Querschnitt entlang der Längenachse kann demnach bspw. rund, oval,

dreieckig, viereckig oder polygonal sein. Lichtemittierende Faser weisen bevorzugte Eigenschaften hinsichtlich ihrer Verwendung auf. So eignen sie sich u.a. für die Anwendung im Bereich der therapeutischen und/oder kosmetischen Phototherapie. Weitere Einzelheiten hierzu sind im Stand der Technik beschrieben (bspw. in US 6538375, US 2003/0099858, Brenndan O'Connor et al. (Adv. Mater. 2007, 19, 3897-3900 und der nicht offengelegte Patentanmeldung EP 10002558.4).

Wenn die organische Elektrolumineszenzvorrichtung mehrere emittierende Schichten enthält, wobei mindestens eine emittierende Schicht das erfindungsgemäße Salz bzw. System aufweist, weisen diese mehreren Schichten bevorzugt insgesamt mehrere Emissionsmaxima zwischen 380 nm und 750 nm auf, so dass insgesamt weiße Emission resultiert, d.h. in den emittierenden Schichten werden verschiedene emittierende Verbindungen verwendet, die fluoreszieren oder phosphoreszieren können. Insbesondere bevorzugt sind drei Schichtsysteme, wobei die drei Schichten blaue, grüne und orange oder rote Emission zeigen, für den prinzipiellen Aufbau siehe zum Beispiel WO 2005/011013.

Die verschiedenen Schichten können im Sinne der Erfindung unterschiedlich aufgetragen werden. Beispielsweise kann in der erfindungsgemäßen Elektrolumineszenzvorrichtung eine oder mehrere Schichten aus Lösung sowie eine oder mehrere Schichten über ein Sublimationsverfahren aufgebracht werden. Dabei werden die Materialien in Vakuumsublimationsanlagen bei einem Druck < $10^{-5}$ mbar, bevorzugt < $10^{-6}$ mbar, besonders bevorzugt < $10^{-7}$ mbar aufgedampft. Ebenfalls ist es möglich, eine oder mehrere Schichten mit OVPD-(Organic Vapor Phase Deposition)-Verfahren oder mit Hilfe einer Trägergassublimation aufzubringen. Dabei werden die Materialien bei einem Druck zwischen $10^{-5}$ mbar und 1 bar aufgebracht. Ein Spezialfall dieses Verfahrens ist das OVJP-(Organic Vapor Jet Printing)-Verfahren, bei dem die Materialien direkt durch eine Düse aufgebracht und so strukturiert werden (z.B. M. S. Arnold et al., Appl. Phys. Lett. 2008, 92, 053301).

Besonders bevorzugt ist jedoch, dass eine oder mehrere Schichten in der organischen Elektrolumineszenzvorrichtung aus Lösung aufgebracht werden, beispielsweise durch Spin-Coating oder mit einem beliebigen Druckverfahren, wie zum Beispiel Siebdruck, Flexodruck oder Offsetdruck. Besonders bevorzugt aber LITI (Light Induced Thermal Imaging, Thermotransferdruck), oder Inkjet-Druck (Tintenstrahldruck). Diese Verfahren sind dem Fachmann generell bekannt und können von ihm ohne Probleme auf organische Elektrolumineszenzvorrichtungen angewandt werden. Für die Anwendung dieser Verfahren ist es besonders bevorzugt, dass das erfindungsgemäße Salz bzw. System in einem polaren Lösungsmittel, wie z.B. Wasser, Acetonitril, Aceton, Tetrahydrofuran, Dimethylformamid, Cyclohexanon oder Dimethylsulfoxid, oder in einem gewöhnlichen Lösungsmittel, wie z. B. Toluol, Chlorbenzol, Anisol, etc., oder einem Lösungsmittelgemisch daraus löslich ist. Auf diese Weise kann eine Miniemulsion hergestellt werden, die aus einer kontinuierlichen Phase und Mikrotropfen besteht. Die kontinuierliche Phase umfasst vorzugsweise ein nicht-polares Lösungsmittel, in dem die Lösung des erfindungsgemäßen Salzes als Nano-Tropfen vorliegen. Vorzugsweise enthält das nicht-polare Lösungsmittel eine Verbindung, welche ein Matrixmaterial für das erfindungsgemäße Salz darstellt. Auf diese Weise kann beispielsweise nach Entfernen der Lösungsmittel eine Schicht hergestellt werden, in der die Emitterverbindungen des erfindungsgemäßen Salzes bzw. Systems als Gastmoleküle in das das Matrixmaterial bildende Verbindunge eingebettet ist.

Die Vorrichtung enthält gewöhnlich eine Kathode und eine Anode (Elektroden). Die Elektroden (Kathode, Anode) werden im Sinne dieser Erfindung so gewählt, dass ihr Potential möglichst gut mit dem Potential der angrenzenden organischen Schicht übereinstimmt, um eine möglichst effiziente Elektronen- bzw. Lochinjektion zu gewährleisten.

Als Kathode sind Metallkomplexe, Metalle mit geringer Austrittsarbeit, Metalllegierungen oder mehrlagige Strukturen aus verschiedenen Metallen bevorzugt, wie beispielsweise Erdalkalimetalle, Alkalimetalle, Hauptgruppenmetalle oder Lathanoide (z. B. Ca, Ba, Mg, Al, In, Mg, Yb, Sm, etc.). Bei mehrlagigen Strukturen können auch zusätzlich zu den genannten Metallen weitere Metalle verwendet werden, die eine relativ hohe Austrittsarbeit aufweisen, wie z. B. Ag, wobei dann in der Regel Kombinationen der Metalle, wie beispielsweise Ca/Ag oder Ba/Ag verwendet werden. Es kann auch bevorzugt sein, zwischen einer metallischen Kathode und dem organischen Halbleiter eine dünne Zwischenschicht eines Materials mit einer hohen Dielektrizitätskonstante einzubringen. Hierfür kommen beispielsweise Alkalimetall- oder Erdalkalimetallfluoride, aber auch die entsprechenden Oxide in Frage (z.B. LiF, $Li_2O$, $BaF_2$, MgO, NaF, etc.). Die Schichtdicke dieser Schicht beträgt bevorzugt zwischen 1 und 10 nm, mehr bevorzugt 2 - 8 nm.

Als Anode sind Materialien mit hoher Austrittsarbeit bevorzugt. Bevorzugt weist die Anode ein Potential größer 4.5 eV vs. Vakuum auf. Hierfür sind einerseits Metalle mit hohem Redoxpotential geeignet, wie beispielsweise Ag, Pt oder Au. Es können andererseits auch Metall/Metalloxid-Elektronen (z. B. Al/Ni/$NiO_x$, Al/$PtO_x$) bevorzugt sein. Für einige Anwendungen muss mindestens eine der Elektroden transparent sein, um entweder die Bestrahlung des organischen Materials (O-SC) oder die Auskopplung von Licht (OLED/PLED, O-LASER) zu ermöglichen. Ein bevorzugter Aufbau verwendet eine transparente Anode. Bevorzugte Anodenmaterialien sind hier leitfähige gemischte Metalloxide. Besonders bevorzugt sind Indium-Zinn-Oxid (ITO) oder Indium-Zink-Oxid (IZO). Bevorzugt sind weiterhin leitfähige, dotierte organische Materialien, insbesondere leitfähige dotierte Polymere.

Die Vorrichtung wird in an sich bekannter Weise je nach Anwendung entsprechend strukturiert, kontaktiert und schließlich hermetisch versiegelt, da sich die Lebensdauer derartiger Vorrichtungen bei Anwesenheit von Wasser und/oder Luft drastisch verkürzt.

Die erfindungsgemäße organische Elektrolumineszenzvorrichtung ist bevorzugt ausgewählt aus der Gruppe, bestehend aus organischen Elektrolumineszenzvorrichtungen (OLEDs), organischen Feld-Effekt-Transistoren (O-FETs), organischen Dünnfilmtransistoren (O-TFTs), organischen lichtemittierenden Transistoren (O-LETs), organischen integrierten Schaltungen (O-ICs), organischen Solarzellen (O-SCs), organischen Feld-Quench-Devices (O-FQDs), lichtemittierenden elektrochemischen Zellen (LECs), organischen Photorezeptoren, "organic plasmon emitting devices" oder organischen Laserdioden (O-Laser). Besonders bevorzugt sind organische Elektrolumineszenzvorrichtungen.

Die erfindungsgemäßen Salze können weiterhin verwendet werden in therapeutischen und kosmetischen Vorrichtungen um Krankheiten zu behandeln und/oder um kosmetische Effekte von Licht bzw. Starhlung zu nutzen.

Ein weiterer Gegenstand der vorliegenden Erfindung bezieht sich daher auf die Verwendung der erfindungsgemäßen Salze, Formulierungen und Vorrichtungen enthaltend die Dalze zur Behandlung, Prophylaxe und Diagnose von Erkrankungen. Noch ein weiterer Gegenstand der vorliegenden Erfindung bezieht sich auf die Verwendung, der erfindungs¬gemäßen Verbindungen und Vorrichtungen enthaltend die Salze zur Behandlung und Prophylaxe kosmetischen Umstände.

Ein weiterer Gegenstand der vorliegenden Erfindung bezieht sich auf die erfindungsgemäßen Salze zu Herstellung von Vorrichtungen zur Therapie, Prophylaxe und/oder Diagnose therapeutischer Erkrankungen.

Dabei sind viele Erkrankungen mit kosmetischen Aspekten assoziiert. So leidet ein Patient mit schwerer Akne in der Gesichtspartie nicht nur an den medizinischen Ursachen und Folgen der Erkrankung, sondern auch an den kosmetischen Begleitumständen.

Phototherapie oder Lichttherapie findet in vielen medizinischen und/oder kosmetischen Bereichen Anwendung. Die erfindungsgemäßen Salze sowie die Vorrichtungen enthaltend diese Salze können daher zur Therapie und/oder Prophylaxe und/oder Diagnose von allen Erkrankungen und/oder in kosmetischen Anwendungen eingesetzt werden, für die der Fachmann die Anwendung von Phototherapie in Betracht zieht. Der Begriff Phototherapie beinhaltet dabei neben der Be¬strahlung auch die photodynamischen Therapie (PDT) sowie das Desinfizieren und Sterilisieren im Allgemeinen. Behandelt werden können mittels Phototherapie oder Lichttherapie nicht nur Menschen oder Tiere, sondern auch jegliche andere Art lebender oder unbelebter Materie. Hierzu gehören, bspw., Pilze, Bakterien, Mikroben, Viren, Eukaryonten, Prokaryonten, Nahrungsmittel, Getränke, Wasser und Trinkwasser.

Der Begriff Phototherapie beinhaltet auch jede Art der Kombination von Lichttherapie und anderen Therapiearten, wie bspw. die Behandlung mit Wirkstoffen. Viele Lichttherapien haben zum Ziel, äußere Partien eines Objektes zu bestrahlen oder zu behandeln, so wie die Haut von Menschen und Tieren, Wunden, Schleimhäute, Auge, Haare, Nägel, das Nagelbett, Zahnfleisch und die Zunge. Die erfindungsgemäße Behandlung oder Bestrahlung kann daneben auch innerhalb eines Objektes durchgeführt werden, um bspw. innere Organe (Herz, Lunge etc.) oder Blutgefäße oder die Brust zu behandeln.

Die erfindungsgemäßen therapeutischen und/oder kosmetischen Anwendungsgebiete sind bevorzugt ausgewählt aus der Gruppe der Hauterkrankungen und Haut-assoziierten Erkrankungen oder Veränderungen bzw. Umstände wie bspw. Psoriasis, Hautalterung, Hautfaltenbildung, Hautverjüngung, vergrößerte Hautporen, Cellulite, ölige/fettige Haut, Follikulitis, aktinische Keratose, precancerose aktinische Keratose, Haut Läsionen, sonnengeschädigte und sonnengestresste Haut, Krähenfüße, Haut Ulkus, Akne, Akne rosacea, Narben durch Akne, Akne Bakterien, Photomodulierung fettiger/öliger Talgdrüsen sowie deren umgebende Gewebe, Ikterus, Neugeborenenikterus, Vitiligo, Hautkrebs, Hauttumore, Crigler Naijar, Dermatitis, atopische Dermatitis, diabetische Hautgeschwüre sowie Desensibilisierung der Haut.

Besonders bevorzugt im Sinne der Erfindung sind die Behandlung und/oder Prophylaxe von Psoriasis, Akne, Cellulite, Hautfaltenbildung, Hautalterung, Ikterus und Vitiligo.

Weitere erfindungsgemäße Anwendungsgebiete für die Zusammensetzungen und/oder Vorrichtungen enthaltend die erfindungsgemäßen Zusammensetzungen sind ausgewählt aus der Gruppe der Entzündungserkrankungen, rheumatoide Arthritis, Schmerztherapie, Behandlung von Wunden, neurologische Erkrankungen und Umstände, Ödeme, Paget's Erkrankung, primäre und metastasierende Tumoren, Bindegewebserkrankungen bzw. -veränderungen, Veränderungen des Kollagens, Fibroblasten und von Fibroblasten stammende Zellspiegel in Geweben von Säugetieren, Bestrahlung der Retina, neovasculare und hypertrophe Erkrankungen, allergische Reaktionen, Bestrahlung der Atemwege, Schwitzen, okulare neovaskulare Erkrankungen, virale Infektionen besonders Infektionen durch Herpes Simplex oder HPV (Humane Papillomviren) zur Behandlung von Warzen und Genitalwarzen.

Besonders bevorzugt im Sinne der Erfindung sind die Behandlung und/oder Prophylaxe von rheumatoider Arthritis, viraler Infektionen, und Schmerzen.

Weitere erfindungsgemäße Anwendungsgebiete für die Salze und/oder Vorrichtungen enthaltend die erfindungsgemäßen Salze sind ausgewählt aus der Winterdepression, Schlafkrankheit, Bestrahlung zur Verbesserung der Stimmung, Linderung von Schmerzen besonders Muskelschmerzen durch bspw. Verspannungen oder Gelenkschmerzen, Beseitigung der Steifheit von Gelenken und das Aufhellen der Zähne (Bleaching).

Weitere erfindungsgemäße Anwendungsgebiete für die Salze und/oder Vorrichtungen enthaltend die erfindungsgemäßen Salze sind ausgewählt aus der Gruppe der Desinfektionen. Mit den erfindungsgemäßen Salze und/oder

mit den erfindungsgemäßen Vorrichtungen können jegliche Art von Objekten (unbelebte Materie) oder Subjekten (lebende Materie wie bspw. Mensch und Tier) zum Zweck der Desinfektion, Sterilisation oder Konservierung behandelt werden. Hierzu zählt, zum Beispiel, die Desinfektion von Wunden, die Reduktion von Bakterien, das Desinfizieren chirurgischer Instrumente oder anderer Gegenstände, das Desinfizieren oder Konservieren von Nahrungs- und Lebensmitteln, von Flüssigkeiten, insbesondere Wasser, Trinkwasser und andere Getränke, das Desinfizieren von Schleim¬häuten und Zahnfleisch und Zähnen. Unter Desinfektion wird hierbei die Reduktion lebender mikrobiologischer Verursacher unerwünschter Effekte, wie Bakterien und Keime, verstanden.

Zu dem Zweck der oben genannten Phototherapie emittieren Vorrich¬tungen enthaltend die erfindungsgemäßen Salze bevorzugt Licht der Wellenlänge zwischen 250 and 1250 nm, besonders bevorzugt zwischen 300 and 1000 nm und insbesondere bevorzugt zwischen 400 and 850 nm.

In einer besonders bevorzugten Ausführungsform der vorliegenden Erfindung werden die erfindungsgemäßen Salze in einer organischen lichtemittierenden Diode (OLED) oder einer organischen lichtemittierenden elektrochemischen Zelle (OLEC) zum Zwecke der Phototherapie eingesetzt. Sowohl die OLED als auch die OLEC können dabei einen planaren oder Fiber- bzw. Faser-artigen Aufbau mit belie¬bigem Querschnitt (z.B. rund, oval, polygonal, quadratisch) mit einem ein- oder mehrschichtigen Aufbau aufweisen. Diese OLECs und/oder OLEDs können in andere Vorrichtungen eingebaut werden, die weitere mechanische, adhäsive und/oder elektronische Bausteine (z.B. Batterie und/oder Steuerungseinheit zur Einstellung der Bestrahlungszeiten, -intensitäten und -wellenlängen) enthalten. Diese Vorrichtungen enthaltend die erfindungsgemäßen OLECs und/order OLEDs sind vorzugsweise ausgewählt aus der Gruppe enthaltend Pflaster, Pads, Tapes, Bandagen, Manschetten, Decken, Hauben, Schlaf¬säcken,Textilien und Stents.

Die Verwendung von den genannten Vorrichtungen zu dem genannten therapeutischen und/oder kosmetischen Zweck ist besonders vorteilhaft gegenüber dem Stand der Technik, da mit Hilfe der erfindungsgemäßen Vorrichtungen unter Verwendung der OLEDs und/oder OLECs homogene Bestrahlungen geringerer Bestrahlungsintensitäten an nahezu jedem Ort und zu jeder Tageszeit möglich sind. Die Bestrahlungen können stationär, ambulant und/oder selbst, d.h., ohne Einleitung durch medizinisches oder kosmetisches Fachpersonal durchgeführt werden. So können, bspw., Pflaster unter der Kleidung getragen werden, so dass eine Bestrahlung auch während der Arbeitszeit, in der Freizeit oder während des Schlafes möglich ist. Auf aufwendige stationäre/ambulante Behandlungen mit kann in vielen Fällen verzichtet bzw. deren Häufigkeit reduziert werden. Die erfindungsgemäßen Vorrichtungen können zum Widergebrauch gedacht sein oder Wegwerfartikel darstellen, die nach ein-, zwei oder dreimaligem Gebrauch entsorgt werden können.

Weitere Vorteile gegenüber dem Stand der Technik sind bspw. eine geringere Wärmeentwicklung und emotionale Aspekte. So werden Neugeborene, die aufgrund einer Gelbsucht (Ikterus) therapiert werden müssen typischerweise mit verbundenen Augen in einem Brutkasten, ohne körperlichen Kontakt zur den Eltern bestrahlt, was eine emotionale Stresssituation für Eltern und Neugeborene darstellt. Mit Hilfe einer erfindungsgemäßen Decke enthaltend die erfindungsgemäßen OLEDs und/oder OLECs kann der emotionale Stress signifikant vermindert werden. Zudem ist eine bessere Temperierung des Kindes durch eine verringerte Wärmeproduktion der erfindungsgemäßen Vorrichtungen gegenüber herkömmlicher Bestrahlungsgeräte möglich.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

**Synthese- und Ausführungsbeispiele:**

Die folgenden Materialen werden in dieser Anmeldung verwendet.

V1 ist ein roter Emitter (DOCI), gekauft bei Lambda Physik, Germany.
V2 ist ein blauer Triplettemitter, synthesiert nach WO 2010/089393;
V3 ist ein grüner Triplettemitter, synthesiert nach WO 2007/006380.
V4 und V5 sind blau fluoreszierende Emitter, Produktname C-2284 und I-6076 bei Invitrogen Corporation.
V6 ist ein grün fluoreszierender Emitter, Produktename F-1907 bei Invitrogen Corporation.
V7 ist eine Infrarot-Absorber, synthetisiert nach WO 2010/095676 A1. Z-907 ist ein Farbstoff von Sigma-Aldrich, miz Produkt-Nr. 703168.
M1 ist eine Matrix-Verbindung für fluoreszierende Emitter, synthetisiert nach WO 2007/065678.
M2 ist eine lösliche Matrix-Verbindung für phosphoreszierende Emitter, synthetisiert nach WO 2005/003253.
E1-E4 sind die erfindungsgemäßen Verbindungen.

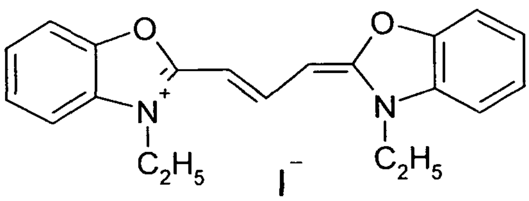

V1

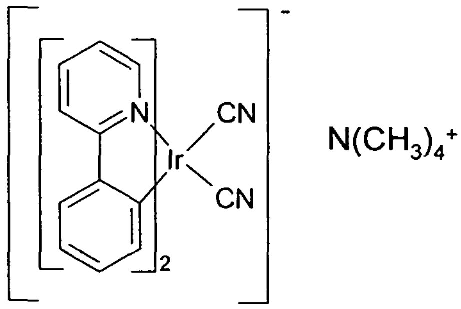

V2

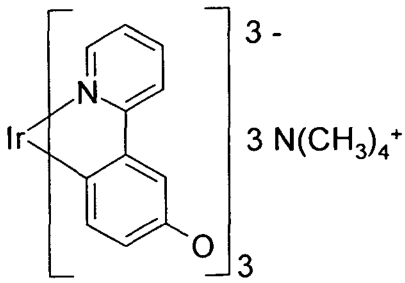

V3

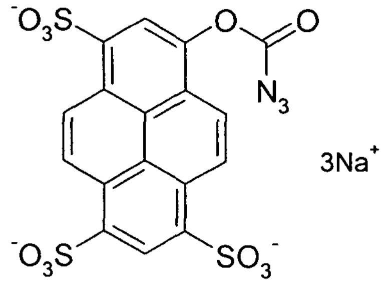

V4

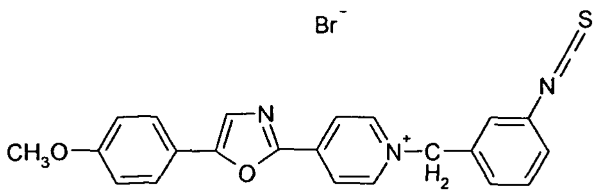

V5

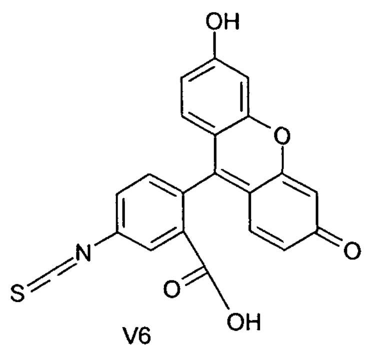

V6

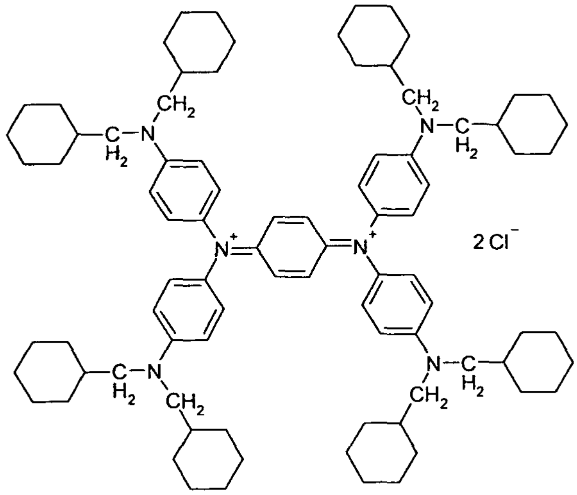

V7

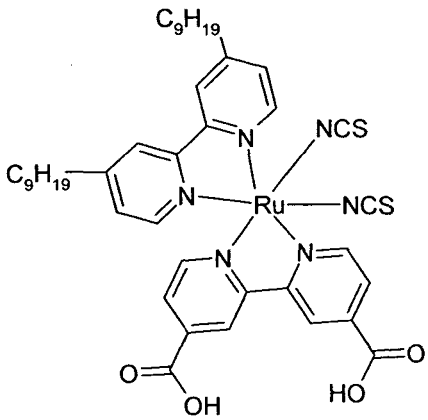

Z-907

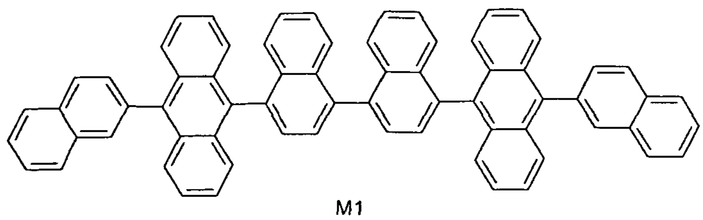

M1

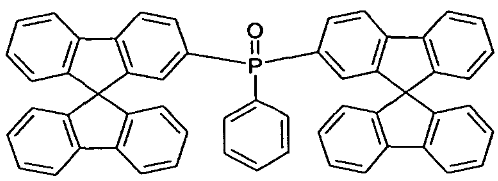

M2

Strukturen der erfindungsgemäßen Verbindungen:

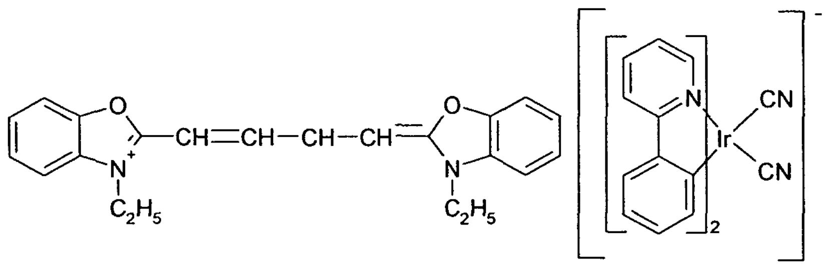

E1

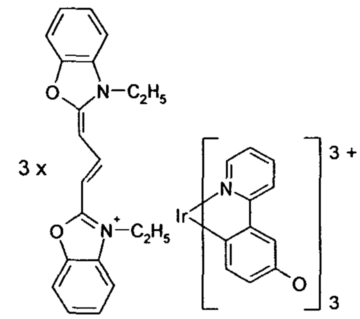

E2

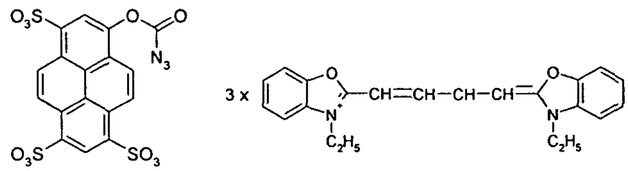

E3

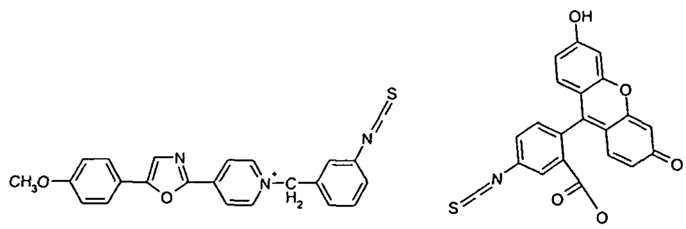

E4

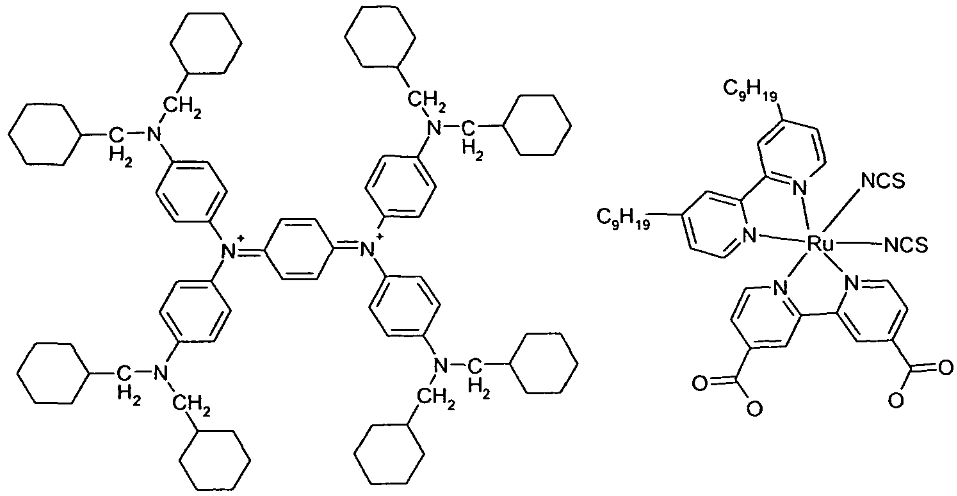

E5

**Beispiel 1**

**Herstellung der Salze E1, E2, E3, und E4**

Eine Lösung von 7.98 g (10 mmol) Tetra-n-butylammonium-bis(cyano-κC)bis[2-(2-pyridinyl-κN)phenyl-κC]iridate(III) [577751-00-9] (Verbindung 1) in 100 ml Acetonitril wird tropfenweise unter gutem Rühren mit einer Lösung von 4.46 g (10 mmol) 3-Ethyl-2-[3-(3-ethyl-2-benzoxazol-inylidene)propenyl]-benzoxazolium iodide [905-96-4] (Verbindung 2) in 100 ml Acetonitril versetzt und dann 30 min. bei Raumtemperatur gerührt. Anschließend konzentriert man die Reaktionsmischung im Vakkum auf ein Volumen von ca. 50 ml, und gibt dann tropfenweise 300 ml eines Wasser-Methanol-Gemisch (1:2, vv). Man rührt noch 1 h bei Raumtemperatur nach, saugt vom ausgefallen Feststoff ab, und kistallisiert diesen dreimal aus Methanol um. Ausbeute: 5.10 g (5.8 mmol), 58%. Reinheit app. 99.5% ($^1$H-NMR).

Analog können folgende Verbindungen hergestellt werden:

| Bsp | Verb. 1 | Verb. 2 | Produkt | Ausbeute |
|---|---|---|---|---|
| E2 | 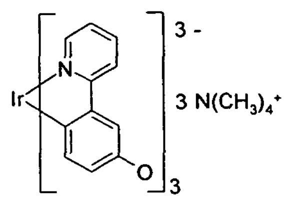 WO 2007/006380 10 mmol | 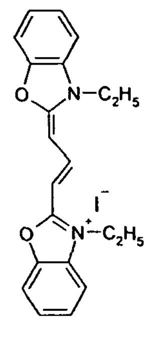 30 mmol | 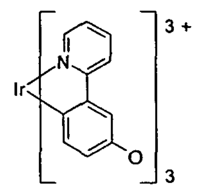 3 x 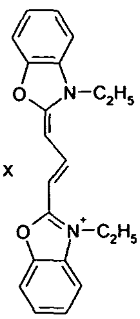 | 61 % |

(fortgesetzt)

| Bsp | Verb. 1 | Verb. 2 | Produkt | Ausbeute |
|---|---|---|---|---|
| E3 | 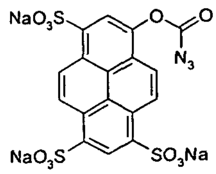 10 mmol | 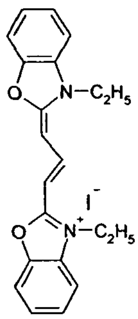 30 mmol | 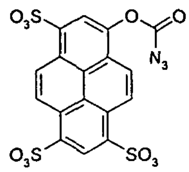 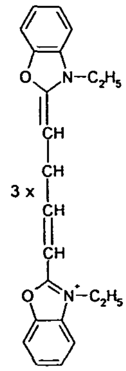 | 45 % |
| E4 | 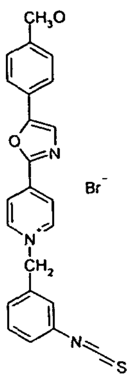 10 mmol | 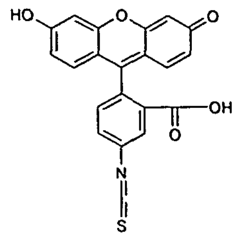 10mmol Zusatz von 10 mmol Triethylamin | 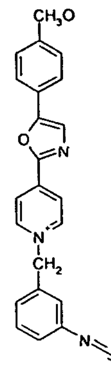 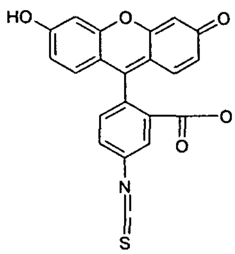 | 39% |
| E5 | V7<br>10mmol | Z-907<br>10mmol | E5 | 28% |

**Beispiel 2**

**Herstellung und Charakterisierung von organischen Elektrolumineszenzvorrichtungen, enthaltend die erfindungsgemäßen Verbindungen E1-E4.**

Die Herstellung einer organischen Leuchtdiode aus Lösung ist in der Literatur bereits vielfach beschrieben (z.B. in der WO 2004/037887 A2). Um die vorliegende Erfindung beispielhaft zu erläutern, wird Tripeltt OLEDs mit verschiedenen Kombinationen von E1-E4 in Matrix durch Spincoating hergestellt.
Eine typische OLED-Vorrichtung hat den Aufbau:

ITO/HIL/Interlayer/EML/Kathode, wobei HIL auch als Pufferschicht genannt wird.

Dazu werden Substrate der Firma Technoprint (Sodalimeglas) verwendet, auf welche die ITO-Struktur (Indium-Zinn-Oxid, eine transparente, leitfähige Anode) aufgebracht wird.

Die Substrate werden im Reinraum mit DI Wasser und einem Detergens (Deconex 15 PF) gereinigt und dann durch eine UV/Ozon-Plasmabehandlung aktiviert. Danach wird ebenfalls im Reinraum als Pufferschicht eine 80 nm Schicht PEDOT (PEDOT ist ein Polythiophen-Derivat (Baytron P VAI 4083sp.) von H. C. Starck, Goslar, das als wässrige Dispersion geliefert wird) durch Spin-Coating aufgebracht. Die benötigte Spinrate hängt vom Verdünnungsgrad und der spezifischen Spincoater-Geometrie ab (typisch für 80 nm: 4500 rpm). Um Restwasser aus der Schicht zu entfernen, werden die Substrate für 10 Minuten bei 180°C auf einer Heizplatte ausgeheizt. Danach werden unter Inertgasatmosphäre (Stickstoff bzw. Argon) zunächst 20 nm einer Interlayer (typischerweise ein lochdominiertes Polymer, hier HIL-012 von Merck KGaA) und dann 80 nm der Emittierendeschichten (EML für Emissive Layer) aus Lösungen (Konzentration 20 g/l in Chlorbenzol, die Kompositionen für die verschiedene EMLs, und denen Konzentrationen sind in Tabelle 1 aufgelistet) aufgebracht. Alle EML Schichten werden bei 60°C mindestens 30 Minuten ausgeheizt. Danach wird die Ba/Al-Kathode aufgedampft (hochreine Metalle von Aldrich, besonders Barium 99,99% (Best-Nr. 474711); Aufdampfanlagen von Lesker o.a., typischer Vakuumlevel 5 x $10^{-6}$mbar). Um vor allem die Kathode vor Luft und Luftfeuchtigkeit zu schützen, wird die Vorrichtung abschließend verkapselt und dann charakterisiert.

Tabelle 1: Die EML Kompositionen in verschiedenen OLEDs

| **Device** | **EML Composition [wt%]** | **Lösungsmittel** | **Konzentration [g/l]** |
|---|---|---|---|
| OLED1 | M2:5%V1 | Chlorbenzol | 20 |
| OLED2 | M2:5%V2 | Chlorbenzol | 20 |
| OLED3 | M2:5%V3 | Chlorbenzol | 20 |
| OLED4 | M2:5%E1 | Chlorbenzol | 20 |
| OLED5 | M2:5%E2 | Chlorbenzol | 20 |
| OLED6 | M1:5%V1 | Chlorbenzol | 20 |
| OLED7 | M1:5%V5 | Chlorbenzol | 20 |
| OLED8 | M1:5%V6 | Chlorbenzol | 20 |
| OLED9 | M1:5%E3 | Chlorbenzol | 20 |
| OLED10 | M1:5%E4 | Chlorbenzol | 20 |

Dazu werden die Vorrichtungen in für die Substratgröße eigens angefertigte Halter eingespannt und mittels Federkontakten kontaktiert. Eine Photodiode mit Augenverlaufsfilter kann direkt auf den Messhalter aufgesetzt werden, um Einflüsse von Fremdlicht auszuschließen.

Typischerweise werden die Spannungen von 0 bis max. 20 V in 0,2 V-Schritten erhöht und wieder erniedrigt. Für jeden Messpunkt wird der Strom durch die Vorrichtung sowie der erhaltene Photostrom von der Photodiode gemessen. Auf diese Art und Weise erhält man die IVL-Daten der Testvorrichtungen. Wichtige Kenngrößen sind die gemessene maximale Effizienz ("Eff." in cd/A) und die für 100 cd/$m^2$ benötigte Spannung $U_{100}$.

Um außerdem die Farbe und das genaue Elektrolumineszenzspektrum der Testvorrichtungen zu kennen, wird nach der ersten Messung nochmals die für 100 cd/$m^2$ benötigte Spannung angelegt und die Photodiode durch einen Spektrum-Messkopf ersetzt. Dieser ist durch eine Lichtleitfaser mit einem Spektrometer (Ocean Optics) verbunden. Aus dem gemessenen Spektrum können die Farbkoordinaten (CIE: Commission International de l'éclairage, Normalbetrachter von 1931) abgeleitet werden.

Die Ergebnisse, die bei Verwendung der Emittern E1 bis E4 in OLEDs erhalten werden, sind in Tabelle 2 zusammengefasst.

Tabelle 2

| Device | Uon M | U(100) [V] | CIE @ 100 cd/$m^2$ | EQE @ Max. Eff. |
|---|---|---|---|---|
| OLED1 | 4.6 | 6.1 | 0.66 / 0.36 | 0.5% |
| OLED2 | 4.3 | 5.9 | 0.19 / 0.31 | 0.9% |
| OLED3 | 3.3 | 4.9 | 0.33 / 0.63 | 1.1% |
| OLED4 | 4.0 | 5.7 | 0.66 / 0.36 | 1.9% |

(fortgesetzt)

| Device | Uon M | U(100) [V] | CIE @ 100 cd/m$^2$ | EQE @ Max. Eff. |
|---|---|---|---|---|
| OLED5 | 3.5 | 5.0 | 0.66 / 0.35 | 2.2% |
| OLED6 | 3.1 | 4.0 | 0.65 / 0.36 | 1.9% |
| OLED7 | 3.9 | 4.7 | 0.18 / 0.33 | 1.3% |
| OLED8 | 3.5 | 4.9 | 0.34 / 0.65 | 2.2% |
| OLED9 | 2.9 | 3.9 | 0.66 / 0.35 | 2.9% |
| OLED10 | 3.0 | 4.6 | 0.33 / 0.66 | 3.8% |

Wie man aus den Ergebnissen erkennen kann, stellen OLED4 und OLED5 eine deutliche Verbesserung gegenüber OLED1-OLED3 hinsichtlich der Effizienz und Betriebsspannung dar. OLED1-OLED3 sind OLEDs mit einzelnen Emittern V1 -V3. Die OLED4 und OLED5 sind OLEDs mit einem erfindungsgemäßen Salz, wobei zwei Emitter so nah in die Matrix dotiert sind, dass der Energietransfer von E1 nach E2 sehr effizient stattfinden kann. Gleiches gilt auch für OLED9 und OLED10 gegenüber OLED6-OLED8.

Ferner kann man auch der Tabelle entnehmen, dass OLED9 & OLED10, worin beide Emitter keine Metallkomplexe-Emitter sind, einen besseren EQE und eine bessere Betriebsspannung im Vergleich zu OLED5 & OLED6 liefern, worin die Salze aus eine Metallkomplex und einem fluoreszierenden Emitter bestehen.

Auf der Grundlage der vorliegenden erfindungsgemäßen technischen Lehre können weitere Optimierungen mittels unterschiedlicher Möglichkeiten realisiert werden ohne dabei erfinderisch zu sein. So kann eine weitere Optimierung bspw. durch die Verwendung andere Matrix oder gemischte Matricen in der gleichen oder einer anderen Konzentration erreicht werden.

**<u>Beispiel 3</u>**

**Absorptionsspektren von V7, Z-907 und E5.**

Die Absorptionspektren von V7, Z-907 und E5 werden in Ethanol Lösung mit einer Konzentration von etwa 0.3 nM gemessen. Im Vergleich zum Z-907, zeigt E5 eine deutlich Verbesserung im nahen Infrarotbereich (650 bis 1000 nm).

## Patentansprüche

1. Salz, bei dem sowohl mindestens ein Kation als auch mindestens ein Anion eine Emitterverbindung oder eine Farbstoffverbindung ist, wobei eine Emitterverbindung eine fluoreszierende Emitterverbindung ist, **dadurch gekennzeichnet, dass** alle Emitterverbindungen Nichtmetallverbindungen sind.

2. Salz nach Anspruch 1, worin alle Emitterverbindungen fluoreszierende Emitterverbindungen sind.

3. Salz nach Anspruch 1 oder 2, das in der Form $[E_1]^{+m}[E_2]^{-m}$ vorliegt, wobei $[E_1]^{+m}$ eine Emitterverbindung mit der Ladung +m ist und $[E_2]^{-m}$ eine Emitterverbindung mit der Ladung -m ist, wobei m gleich 1 oder 2 ist.

4. Salz nach Anspruch 3, worin m gleich 1 ist.

5. Salz nach einem oder mehreren der Ansprüche 1 bis 4, das in der Form $[E_3]^k[E_1]^p[E_2]^k$ vorliegt, wobei $[E_1]^p$ eine Emitterverbindung mit der Ladung p ist und $[E_2]^k$ und $[E_3]^k$ Emitterverbindungen mit der Ladung k sind, wobei und p+2k=0 ist, und vorzugsweise k gleich $\pm1$ oder $\pm2$.

6. Salz nach Anspruch 5, worin $[E_2]^k$ und/oder $[E_3]^k$ (eine) fluoreszierende Emitterverbindung ist/sind.

7. Salz nach Anspruch 5 oder 6, worin k gleich 1 ist.

8. Salz nach einem oder mehreren der Ansprüche 1 bis 8, worin die Emissionsbande einer Emitterverbindung in einem Wellenlängenbereich liegt, der mit dem Wellenlängenbereich der Absorptionsbande einer anderen Emitterverbindung überlappt.

**9.** Salz nach einem oder mehreren der Ansprüche 5 bis 7, worin das Maximum der Emissionsbande von $[E_1]^p$ im Wellenlängenbereich von blauem Licht liegt, und die Maxima der Emissionsbanden von $[E_2]^k$ und $[E_3]^k$ im Wellenlängenbereich von grünem oder rotem Licht liegen.

**10.** Verfahren zur Herstellung eines Salzes nach einem oder mehreren der Ansprüche 1 bis 9 umfassend die folgenden Schritte:

(a) Bereitstellen einer Mehrzahl von Salzen, wobei jedes Salz nur eine Emitterverbindung als Kation oder Anion aufweist und wobei mindestens eine Emitterverbindung eine positive Ladung und mindestens eine Emitterverbindung eine negative Ladung aufweist;
(b) jeweiliges Lösen der bereitgestellten Salze in einem polaren Lösungsmittel, so dass entsprechend der Anzahl der Salze eine Mehrzahl von Lösungen vorliegt;
(c) Mischen der Lösungen;
(d) Kristallisieren eines Salzes, das die mindestens eine Emitterverbindung mit der positiven Ladung als ein Kation und die mindestens eine Emitterverbindung mit der negativen Ladung als ein Anion enthält.

**11.** Verfahren nach Anspruch 10, worin in Schritt (a) zwei Salze $[E_1]^+Y^-$und $[E_2]^-X^+$ bereitgestellt werden, so dass in Schritt (d) ein Salz $[E_1]^+[E_2]^-$ erhalten wird, wobei $Y^-$ und $X^+$ entsprechende Gegenionen sind, die gemeinsam ein Salz $X^+Y^-$ bilden, welches leichter löslich ist als $[E_1]^+[E_2]^-$.

**12.** Verfahren nach Anspruch 11, worin in Schritt (a) drei Salze $[E_1]^{2+}[2Y]^-$, $[E_2]^-[X]^+$ und $[E_3]^-[X]^+$ bereitgestellt werden, so dass in Schritt (d) ein Salz $[E_3]^-[E_1]^{2+}[E_2]^-$ erhalten wird, wobei $Y^-$ und $X^+$ entsprechende Gegenionen sind, die gemeinsam ein Salz $X^+Y^-$bilden, welches leichter löslich ist als $[E_3]^-[E_1]^{2+}[E_2]^-$.

**13.** Verwendung eines Salzes gemäß einem oder mehreren der Ansprüche 1 bis 9 in einer elektronischen Vorrichtung.

**14.** Organische Elektrolumineszenzvorrichtung enthaltend ein Salz gemäß einem oder mehreren der Ansprüche 1 bis 9, bevorzugt ausgewählt aus organischen Leuchtdioden (OLEDs), organischen licht-emittierenden elektrochemischen Zellen (OLECs) und organischen lichtemittierenden Transistoren (OLETs).

**15.** Elektronische Vorrichtung enthaltend ein Salz gemäß einem oder mehreren der Ansprüche 1 bis 9, bevorzugt ausgewählt aus der Gruppe bestehend aus organischen integrierten Schaltungen (O-ICs), organischen Feld-Effekt-Transistoren (O-FETs), organischen Dünn-filmtransistoren (O-TFTs), organischen Solarzellen (O-SCs), farbstoffsensibilisierten organischen Solarzellen (DSSCs), organischen optischen Detektoren, organischen Photorezeptoren, organischen Feld-Quench-Devices (O-FQDs), organischen Laserdioden (O Laser) und "organic plasmon emitting devices".

**16.** Formulierung, insbesondere eine Lösung, Dispersion oder Emulsion, enthaltend ein Salz nach einem oder mehreren der Ansprüche 1 bis 9 gelöst, dispergiert oder emulgiert in mindestens einem Lösungsmittel.

**17.** Organische Elektrolumineszenzvorrichtung gemäß Anspruch 14 zur Verwendung in der Medizin zur Phototherapie.

**18.** Verwendung einer organischen Elektrolumineszenzvorrichtung nach Anspruch 14 in der Kosmetik.

## Claims

**1.** Salt in which both at least one cation and also at least one anion is an emitter compound or a dye compound, where one emitter compound is a fluorescent emitter compound, **characterised in that** all emitter compounds are non-metal compounds.

**2.** Salt according to Claim 1, in which all emitter compounds are fluorescent emitter compounds.

**3.** Salt according to Claim 1 or 2, which is in the form $[E_1]^{+m}[E_2]^{-m}$, where $[E_1]^{+m}$ is an emitter compound having the charge +m and $[E_2]^{-m}$ is an emitter compound having the charge -m, where m is equal to 1 or 2.

**4.** Salt according to Claim 3, in which m is equal to 1.

5. Salt according to one or more of Claims 1 to 4, which is in the form $[E_3]^k[E_1]^p[E_2]^k$, where $[E_1]^p$ is an emitter compound having the charge p and $[E_2]^k$ and $[E_3]^k$ are emitter compounds having the charge k, where p+2k=0, and k is preferably equal to $\pm 1$ or $\pm 2$.

6. Salt according to Claim 5, in which $[E_2]^k$ and/or $[E_3]^k$ is (are) (a) fluorescent emitter compound.

7. Salt according to Claim 5 or 6, in which k is equal to 1.

8. Salt according to one or more of Claims 1 to 7, in which the emission band of an emitter compound is in a wavelength range which overlaps with the wavelength range of the absorption band of another emitter compound.

9. Salt according to one or more of Claims 5 to 7, in which the maximum of the emission band of $[E_1]^p$ is in the wavelength region of blue light, and the maxima of the emission bands of $[E_2]^k$ and $[E_3]^k$ are in the wavelength region of green or red light.

10. Process for the preparation of a salt according to one or more of Claims 1 to 9 comprising the following steps:

(a) provision of a multiplicity of salts, where each salt contains only one emitter compound as cation or anion and where at least one emitter compound has a positive charge and at least one emitter compound has a negative charge;
(b) respective dissolution of the salts provided in a polar solvent, giving a multiplicity of solutions corresponding to the number of salts;
(c) mixing of the solutions;
(d) crystallisation of a salt which contains the at least one emitter compound having the positive charge as a cation and the at least one emitter compound having the negative charge as an anion.

11. Process according to Claim 10, in which two salts $[E_1]^+Y^-$ and $[E_2]^-X^+$ are provided in step (a), so that a salt $[E_1]^+[E_2]^-$ is obtained in step (d), where $Y^-$ and $X^+$ are corresponding counterions which together form a salt $X^+Y^-$ which is more readily soluble than $[E_1]^+[E_2]^-$.

12. Process according to Claim 11, in which three salts $[E_1]^{2+}[2Y]^-$, $[E_2]^-[X]^+$ and $[E_3]^-[X]^+$ are provided in step (a), so that a salt $[E_3]^-[E_1]^{2+}[E_2]^-$ is obtained in step (d), where $Y^-$ and $X^+$ are corresponding counterions which together form a salt $X^+Y^-$ which is more readily soluble than $[E_3]^-[E_1]^{2+}[E_2]^-$.

13. Use of a salt according to one or more of Claims 1 to 9 in an electronic device.

14. Organic electroluminescent device containing a salt according to one or more of Claims 1 to 9, preferably selected from organic light-emitting diodes (OLEDs), organic light-emitting electrochemical cells (OLECs) and organic light-emitting transistors (OLETs).

15. Electronic device containing a salt according to one or more of Claims 1 to 9, preferably selected from the group consisting of organic integrated circuits (O-ICs), organic field-effect transistors (O-FETs), organic thin-film transistors (O-TFTs), organic solar cells (O-SCs), dye-sensitised organic solar cells (DSSCs), organic optical detectors, organic photoreceptors, organic field-quench devices (O-FQDs), organic laser diodes (O-lasers) and "organic plasmon emitting devices".

16. Formulation, in particular a solution, dispersion or emulsion, comprising a salt according to one or more of Claims 1 to 9 dissolved, dispersed or emulsified in at least one solvent.

17. Organic electroluminescent device according to Claim 14 for use in medicine for phototherapy.

18. Use of an organic electroluminescent device according to Claim 14 in the cosmetics field.

## Revendications

1. Sel dans lequel à la fois au moins un cation et également au moins un anion sont un composé émetteur ou un composé de colorant, où un composé émetteur est un émetteur fluorescent, **caractérisé en ce que** tous les composés émetteurs sont des composés non métalliques.

**2.** Sel selon la revendication 1, dans lequel tous les composés émetteurs sont des composés émetteurs fluorescents.

**3.** Sel selon la revendication 1 ou 2, lequel est sous la forme $[E_1]^{+m}[E_2]^{-m}$, où $[E_1]^{+m}$ est un composé émetteur qui comporte la charge +m et $[E_2]^{-m}$ est un composé émetteur qui comporte la charge -m, où m est égal à 1 ou 2.

**4.** Sel selon la revendication 3, dans lequel m est égal à 1.

**5.** Sel selon une ou plusieurs des revendications 1 à 4, lequel est sous la forme $[E_3]^k[E_1]^p[E_2]^k$, où $[E_1]^p$ est un composé émetteur qui comporte la charge p et $[E_2]^k$ et $[E_3]^k$ sont des composés émetteurs qui comportent la charge k, où p+2k=0, et k est de préférence égal à $\pm 1$ ou $\pm 2$.

**6.** Sel selon la revendication 5, dans lequel $[E_2]^k$ et/ou $[E_3]^k$ est (sont) (a) un composé émetteur fluorescent.

**7.** Sel selon la revendication 5 ou 6, dans lequel k est égal à 1.

**8.** Sel selon une ou plusieurs des revendications 1 à 7, dans lequel la bande d'émission d'un composé émetteur est dans une plage de longueurs d'onde qui chevauche la plage de longueurs d'onde de la bande d'absorption d'un autre composé émetteur.

**9.** Sel selon une ou plusieurs des revendications 5 à 7, dans lequel le maximum de la bande d'émission de $[E_1]^p$ est dans la région des longueurs d'onde de la lumière bleue, et les maximums des bandes d'émission de $[E_2]^k$ et $[E_3]^k$ sont dans la région des longueurs d'onde de la lumière verte ou rouge.

**10.** Procédé pour la préparation d'un sel selon une ou plusieurs des revendications 1 à 9 comprenant les étapes qui suivent :

(a) fourniture d'une multiplicité de sels, où chaque sel contient seulement un composé émetteur tel qu'un cation ou qu'un anion et où au moins un composé émetteur comporte une charge positive et au moins un composé émetteur comporte une charge négative ;
(b) dissolution respective des sels fournis dans un solvant polaire, d'où l'obtention d'une multiplicité de solutions correspondant au nombre de sels ;
(c) mélange des solutions ;
(d) cristallisation d'un sel qui contient l'au moins un composé émetteur qui comporte la charge positive tel qu'un cation et de l'au moins un composé émetteur qui comporte la charge négative tel qu'un anion.

**11.** Procédé selon la revendication 10, dans lequel deux sels $[E_1]^+Y^-$ et $[E_2]^-X^+$ sont fournis au niveau de l'étape (a), de telle sorte qu'un sel $[E_1]^+[E_2]^-$ soit obtenu au niveau de l'étape (d), où $Y^-$ et $X^+$ sont des contre-ions correspondants qui forment ensemble un sel $X^+Y^-$qui est davantage aisément soluble que $[E_1]^+[E_2]^-$.

**12.** Procédé selon la revendication 11, dans lequel trois sels $[E_1]^{2+}[2Y]^-$, $[E_2]^-[X]^+$ et $[E_3]^-[X]^+$ sont fournis au niveau de l'étape (a), de telle sorte qu'un sel $[E_3]^-[E_1]^{2+}[E_2]^-$ soit obtenu au niveau de l'étape (d), où $Y^-$ et $X^+$ sont des contre-ions correspondants qui forment ensemble un sel $X^+Y^-$ qui est davantage aisément soluble que $[E_3]^-[E_1]^{2+}[E_2]^-$.

**13.** Utilisation d'un sel selon une ou plusieurs des revendications 1 à 9 dans un dispositif électronique.

**14.** Dispositif électroluminescent organique qui contient un sel selon une ou plusieurs des revendications 1 à 9, de préférence sélectionné parmi les diodes émettrices de lumière organiques (OLED), les cellules électrochimiques à émission de lumière organiques (OLEC) et les transistors à émission de lumière organiques (OLET).

**15.** Dispositif électronique qui contient un sel selon une ou plusieurs des revendications 1 à 9, de préférence sélectionné parmi le groupe qui est constitué par les circuits intégrés organiques (O-IC), les transistors à effet de champ organiques (O-FET), les transistors à film mince organiques (O-TFT), les cellules solaires organiques (O-SC), les cellules solaires organiques sensibilisées par colorant (DSSC), les détecteurs optiques organiques, les photorécepteurs organiques, les dispositifs à extinction de champ organiques (O-FQD), les diodes laser organiques (O-laser) et les "dispositifs à émission de plasmons organiques".

**16.** Formulation, en particulier une solution, une dispersion ou une émulsion, comprenant un sel selon une ou plusieurs des revendications 1 à 9 dissout, dispersé ou émulsifié dans au moins un solvant.

**17.** Dispositif électroluminescent organique selon la revendication 14 pour une utilisation en médecine pour la photothérapie.

**18.** Utilisation d'un dispositif électroluminescent organique selon la revendication 14 dans le domaine des cosmétiques.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9013148 A1
- US 2010059740 A1
- WO 2006122630 A
- WO 2008006449 A
- WO 2007140847 A
- DE 102009005746
- US 20070252517 A1
- US 4769292 A
- US 6020078 A
- US 11097352 B
- US 5121029 A
- US 5130603 A
- US 20070092753 A1
- DE 102008035413
- US 2009000658 A
- WO 2009107100 A
- WO 2009098643 A
- US 6245988 B
- WO 2010055471 A
- JP 2010084003 A
- EP 1622178 A
- WO 9850393 A
- WO 9529924 A
- WO 9404497 A
- WO 9214741 A
- WO 9116719 A
- WO 007065 A
- WO 0141512 A
- WO 0202714 A
- WO 0215645 A
- EP 1191613 A
- EP 1191612 A
- EP 1191614 A
- WO 2005033244 A
- WO 2005055248 A1
- EP 676461 A
- WO 2009069566 A
- WO 04081017 A
- WO 2004058911 A
- WO 2005084081 A
- WO 2005084082 A
- WO 2006048268 A
- WO 2006117052 A
- WO 2008145239 A
- US 20080193796 A1
- WO 2005039246 A
- US 20050069729 A
- JP 2004288381 A
- EP 1205527 A
- WO 2008086851 A
- EP 1617710 A
- EP 1617711 A
- EP 1731584 A
- JP 2005347160 A
- WO 2004093207 A
- DE 102008033943
- WO 2005003253 A
- WO 2007137725 A
- WO 2005111172 A
- DE 102008017591
- DE 102008036982
- WO 2007063754 A
- WO 2008056746 A
- DE 102009023155
- DE 102009031021
- DE 102009022858
- US 20070134514 A1
- US 20050249976 A
- US 20070128467 A
- US 004209115 A
- US 20040209116 A
- US 20070087219 A
- EP 652273 A
- US 6538375 B
- US 20030099858 A
- EP 10002558 A
- WO 2005011013 A
- WO 2010089393 A
- WO 2007006380 A
- WO 2010095676 A1
- WO 2007065678 A
- WO 2004037887 A2


### In der Beschreibung aufgeführte Nicht-Patentliteratur


- **KAWAMURA, Y. ; YANAGIDA, S. ; FORREST, S.R.** Energy transfer in polymer electro phosphorescent light emitting device with single and multiple doped luminescent layers. *J. Appl. Phys.,* 2002, vol. 92 (1), 87-93
- **C.H.CHEN et al.** Recent developments in organic electroluminescent materials. *Macromol. Symp.,* 1997, vol. 125, 1-48

- Recent progress of molecular organic electroluminescent materials and devices. *Mat. Sci. and Eng. R,* 2002, vol. 39, 143-222
- **T. FÖRSTER.** Zwischenmolekulare Energiewanderung und Fluoreszenz. *Ann. Physic.,* 1948, vol. 437, 55
- **D. L. DEXTER.** *J. Chem. Phys.,* 1953, vol. 21, 836
- *The Journal of Physical Chemistry C,* 2009, vol. 113, 2966-2973
- **BALDO et al.** *Nature,* 2000, vol. 403 (6771), 750-753
- **SEGAL et al.** *Nat.Mater.,* 2007, vol. 6 (5), 374-378
- **M. FISCHER et al.** *Angew. Chem., Int. Ed.,* 1999, vol. 38, 885
- **CROWLEY, J. D. ; TEAGUE, G. S. JR. ; LOWE, J. W. JR.** *Journal of Paint Technology,* 1966, vol. 38 (496), 296
- **W.H. ELLIS.** Solvents. Federation of Societies for Coatings Technology, 1986, 9-10
- **LANDFESTER.** *Annu. Rev, Mater. Res.,* vol. 36 (06), 231
- **BRENNDAN O'CONNOR et al.** *Adv. Mater.,* 2007, vol. 19, 3897-3900
- **M. S. ARNOLD et al.** *Appl. Phys. Lett.,* 2008, vol. 92, 053301